(19) Europäisches Patentamt
European Patent Office
Office européen des brevets

(11) **EP 2 262 467 B1**

(12) **EUROPEAN PATENT SPECIFICATION**

(45) Date of publication and mention
of the grant of the patent:
**20.09.2017  Bulletin 2017/38**

(21) Application number: **09716792.8**

(22) Date of filing: **05.02.2009**

(51) Int Cl.:
*A61K 8/25* (2006.01)        *A61K 8/02* (2006.01)
*B01J 13/20* (2006.01)       *A61Q 1/10* (2006.01)
*A61K 8/70* (2006.01)        *A61K 8/895* (2006.01)
*A61K 8/97* (2017.01)        *A61K 8/11* (2006.01)
*A61K 8/19* (2006.01)        *A61K 8/58* (2006.01)
*A61K 8/81* (2006.01)

(86) International application number:
**PCT/US2009/033135**

(87) International publication number:
**WO 2009/111128 (11.09.2009 Gazette 2009/37)**

(54) **COSMETIC COMPOSITIONS FOR IMPARTING SUPERHYDROPHOBIC FILMS**

KOSMETISCHE ZUSAMMENSETZUNGEN ZUR ERZEUGUNG VON SUPERHYDROPHOBEN FILMÜBERZÜGEN

COMPOSITIONS COSMÉTIQUES DESTINÉES À CRÉER DES FILMS SUPERHYDROPHOBES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority:  **04.03.2008  US 33536**

(43) Date of publication of application:
**22.12.2010  Bulletin 2010/51**

(73) Proprietor: **Avon Products, Inc.**
**New York, NY 10017 (US)**

(72) Inventors:
• **RANADE, Rahul, A.**
**Emerson**
**New Jersey 07630 (US)**

• **GARRISON, Mark, S.**
**Suffern**
**New York 10901 (US)**

(74) Representative: **Dr. Weitzel & Partner**
**Patent- und Rechtsanwälte mbB**
**Friedenstrasse 10**
**89522 Heidenheim (DE)**

(56) References cited:
**WO-A1-2007/040517        JP-A- 2001 181 136**
**US-A1- 2004 033 451        US-B1- 6 534 044**
**US-B2- 6 683 126**

**Description**

RELATED APPLICATIONS

This application is a non-provisional counterpart to and claims priority to pending U.S. Serial No. 61/033,536, filed on 4 March 2008, whichis pending.

FIELD OF INVENTION

The present invention relates to methods and compositions as defined in the claims for imparting a hydrophobic film on a surface. More specifically, the invention relates to cosmetic compositions and methods for forming a super-hydrophobic film on the skin or hair.

BACKGROUND OF THE INVENTION

The leaf of the lotus plant exhibits remarkable water-repellency and self-cleaning properties. Although lotus plants prefer to grow in muddy rivers and lakes, the leaves and flowers remain clean and are essentially non-wettable. The lotus plant achieves this effect by producing leaves and flowers with extremely hydrophobic surfaces. When the leaves come in contact with water, the water droplets contract into substantially spherical beads which roll off the surface, sweeping away any particles of dirt they encounter.

On extremely hydrophilic surfaces, a water droplet will completely spread and provide an effective contact angle of essentially 0°. This occurs for surfaces that have a large affinity for water, including materials that absorb water, On many hydrophilic surfaces, water droplets will exhibit contact angles of about 10° to about 30°. In contrast, on hydrophobic surfaces, which are incompatible with water, larger contact angles are observed, typically in the range of about 70° to about 90° and above. Some very hydrophobic materials, for example, Teflon™, which is widely regarded as a benchmark of hydrophobic surfaces, provides a contact angle with water of as high as 120°-130°,

Against this background, it is remarkable that the lotus leaf can produce a contact angle with water of about 160°, which is substantially more hydrophobic that Teflon™. The lotus leaf is thus an example of a "super-hydrophobic" surface, For the present purposes, a super-hydrophobic surface may be said to be one which provides a contact angle with water of greater than about 140°. This effect is believed to arise due to the three-dimensional surface structure of the leaf wherein wax crystals self-organize to provide roughness on a nano- or micro-meter scale. The hydrophobic surface protuberances reduce the effective surface contact area with water and thus prevent adhesion and spreading of the water over the leaf.

The discovery of the aforementioned properties of the lotus leaf and elucidation of its mechanism has led to a variety of engineered super-hydrophobic surfaces. Such super-hydrophobic surfaces have water contact angles ranging from 140° to nearly 180°. Such surfaces are extremely difficult to wet. On these surfaces, water droplets simply rest on the surface, without actually wetting to any significant extent. Superhydrophobic surfaces have been obtained in a variety of ways. Some of these very hydrophobic materials are found in nature. Other superhydrophobic materials are made synthetically, sometimes as mimics of natural materials,

U.S. Patent 6,683,126 describes a coating composition for producing difficult to wet surfaces comprising a finely divided powder, where the particles are porous and have a hydrophobic surface, combined with a film forming binder such that the ratio of the powder to the binder is 1:4.

U.S. Patent 6,852,389 describes the process of production of superhydrophobic materials for self cleaning applications.

U.S. Patent 6,946,170 describes a self cleaning display device.

U.S. Patent 7,056,845 describes a method for the application of a finishing layer which is water repellant for use in finishing of textiles, fabrics and tissues,

U.S. Patent 6,800,354 describes process of production of self cleaning substrates of glass, ceramic, and plastics.

U.S. Patent No. 5,500,216 describes a method of reducing drag through water by applying a film of rough particles of hydrophobic metal oxides where the particles have a distribution of two different size ranges. Water-repellent cosmetics used for make-up cosmetics, such as foundation, an eye-shadow and rouge, are disclosed in JP2001181136.

While hydrophobic or super-hydrophobic materials have been described above, there remains a need for hydrophobic or super-hydrophobic materials in cosmetic compositions to impart superhydrophobic films on surfaces such as skin, hair, or nails. Conventional water-proof or water-resistant cosmetic compositions are generally made from oil-in-water or water-in-oil emulsions. Water-in-oil emulsions tend to have an oily feel, thus limiting their use. The conventional approach to formulating water-proof or water-resistant cosmetic compositions relies on the use of hydrophobic film formers (e.g. waxes) to form a water-resistant barrier. Such conventional cosmetics are at best hydrophobic, as opposed to the super-hydrophobic films of the present invention.

EP 2 262 467 B1

Conventional water-proof or water-resistant topical compositions are not super-hydrophobic primarily because they lack nano-scale or micro-scale surface roughness. In the absence of roughness on the nano- or micro-meter scale, smooth films made of currently known hydrophobic materials exhibit contact angles that are not in the super-hydrophobic range, i.e., they are less than 140°. It would be desirable to provide cosmetic films which impart super-hydrophobic films for improving water repellency, self-cleaning properties, and long-wear properties.

It is therefore an object of the invention to provide cosmetic compositions for application to the skin, hair, or nails which form a super-hydrophobic film thereon. It is a further object of the invention to provide methods for imparting superhydrophobic films to skin, hair, and nails to achieve water-resistant, self-cleaning and/or long-wear properties.

SUMMARY OF THE INVENTION

In accordance with the foregoing objectives and others, the present invention provides compositions and methods as defined in the claims for forming super-hydrophobic films on a surface, preferably a biological integurnent, such as skin, nail, or hairs.

In the broadest aspect of the invention, compositions are provided for rendering a surface superhydrophobic comprising:

(a) one or more hydrophobic film formers,

(b) a combination of hydrophobic pigments comprising: (i) one or more hydrophobically-modified iron oxide pigments; and (ii) a carbon black powder; wherein the weight ratio of said one or more hydrophobically-modified iron oxide pigments to said carbon black powder is being between 1:10 to 10:1, and wherein the one or more hydropobically-modified iron oxide pigment is selected from the group consisting of alkylsilane-treated iron oxide pigments, and perfluoroalkylsilane-treated iron oxide pigments and mixtures thereof; wherein the weight ratio of said one or more hydrophobic film formers to said combination of hydrophobic pigments is from 1:10 to 5:1; and wherein the aggregate weight percentage of all non-volatile water-soluble or water-dispersible organic constituents in said composition is less than 15%, based on the entire weight of the composition; wherein the composition is capable of providing a film on a surface which, after evaporation of any volatile constituents present, is characterized by a contact angle with water greater than about 140°.

According to one embodiment or disclosure, the hydrophobically-modified iron oxide pigments may comprise a surface treatment selected from, without limitation, the group consisting of alkyl, allyls, vinyls, aryl, alkyl-aryl, aryl-alkyl, silanes, silicones, dimethicones, fatty acids, polymeric silanes, polyurethanes, epoxies, and fluoro- or perfluoro- derivatives thereof.

Also provided are cosmetic compositions for imparting a superhydrophobic film on an integument, comprising:

(a) one or more hydrophobic film formers, and

(b) one or more alkylsilane-treated iron oxide pigments, the iron oxide pigments having a ratio of percent surface treatment to mean particle size greater than about 2.3;

wherein the weight ratio of the one or more hydrophobic film formers to the one or more alkylsilane-treated iron oxide pigments is from about 1:10 to about 5:1, typically from about 1;5 to about 2:1, more typically from about 1:2 to about 1:1; and wherein the aggregate weight percentage of all non-volatile water-soluble or water-dispersible organic constituents in the composition is less than 15%, typically less than 10%, preferably less than and more preferably less than 2%, based on the entire weight of the composition; the weight percentage of all polyols in the aggregate preferably being below 1%;

the composition being capable of providing a film on a surface which, after evaporation of volatile constituents, is characterized by a contact angle with water greater than about 140°

In some embodiments, the one or more alkylsilane-treated iron oxide pigments will comprise a trialkyoxyalkylsilane treated iron oxide pigment, and in particular a triethoxycaprylylsilane treated iron oxide pigment. The pigment will preferably have a ratio of percent surface treatment to mean particle size greater than about 2.5.

Cosmetic composition for imparting a superhydrophobic film on an integument are also provided comprising:

(a) one or more hydrophobic film formers; and

(b) one or more fluorosilane-treated iron oxide pigments, said iron oxide pigments having a ratio of percent surface treatment to mean particle size greater than about 1.2;

3

wherein the weight ratio of the one or more hydrophobic film formers to the one or more fluorosilane-treated iron oxide pigments is from about 1:10 to about 5: is typically from about 1:5 to about 2:1, more typically from about 1:2 to about 1:1; and wherein the aggregate weight percentage of all non-volatile water-soluble or water-dispersible organic constituents in the composition is less than 15%, typically Less than 10%, preferably less than 5%, and more preferably less than 2%, based on the entire weight of the composition; the weight percentage of all polyols in the aggregate preferably being below 1%;

the composition being capable of providing a film on a surface which, after evaporation of volatile constituents, is characterized by a contact angle with water greater than about 140°.

In some embodiments, the fluorosilane-treated pigments include one or more fluoroalkylsilane-tieated iron oxide pigment, For example, a perfluoroalkyl trialkyoxysilane treated iron oxide pigment, such as a Perfluorooctyl Triethoxysilane treated iron oxide pigment is suitable. The fluorosilane-treated iron oxide pigment may have a ratio of percent surface treatment to mean particle size greater than about 1.5.

Cosmetic compositions for imparting a superhydrophobic film on an integument are also provided comprising;

(a) one or more hydrophobic film formers; and
(b) carbon black; the carbon black preferably having a mean particle size between about 0.01 $\mu$m and about 1 $\mu$m and/or a surface area between about 200 and about 260 m$^2$/g;

wherein the weight ratio of the one or more hydrophobic film formers to carbon black powder is from about 1:10 to about 5: 1, typically from about 1:5 to about 2;1, more typically from about 1:2 to about 1:1; and wherein the aggregate weight percentage of all non-volatile water-soluble or water-dispersible organic constituents in the composition is less than 15%, typically less than 10%, preferably less than 5%, and more preferably less than 2%, based on the entire weight of the composition; the weight percentage of all polyols in the aggregate preferably being below 1 %;

the composition being capable of providing a film on a surface which, after evaporation of volatile constituents, is characterized by a contact angle with water greater than about 140°, more typically greater than about 145°, and preferably greater than about 148°

In a particularly unexpected aspect of the invention or disclosure, synergies between hydrophobically-modified iron oxide pigments and carbon black pigments are observed. According to this aspect, cosmetic compositions for imparting a superhydrophobic film on an integument are provided comprising:

(a) one or more hydrophobic film formers; and
(b) a combination of hydrophobic pigments comprising: (i) one or more hydrophobically-modified iron oxide pigments; and (ii) carbon black; the weight ratio of said one or more hydrophobically-modified iron oxide pigments to said carbon black being between about 1:10 to about 10:1:

wherein the weight ratio of said one or more hydrophobic film formers to said combination of hydrophobic pigments is from about 1:10 to about 5:1, typically from about 1:5 to about 2:1, more typically from about 1:2 to about 1:1; and wherein the aggregate weight percentage of all non-volatile water-soluble or water-dispersible organic constituents in said composition is less than 15%, typically less than 10%, preferably less than 5%, and more preferably less than 2%, based on the entire weight of the composition; the weight percentage of all polyols in the aggregate preferably being below 1%; the composition being capable of providing a film on a surface which, after evaporation of volatile constituents, is characterized by a contact angle with water greater than about 140°, more typically greater than about 145°, preferably greater than about 148°, and more preferably greater than about 150°.

The carbon black powder preferably has a mean particle size between about 0.01 $\mu$m and about 1 $\mu$m and/or a surface area between about 200 and about 260 m$^2$/g.

The hydrophobically-modified iron oxide pigments according to this embodiment may be any iron oxide pigments, including for example alkylsilane-treated iron oxide pigments and a perfluoroalkylsilane-treated iron oxide pigments. Trialkyoxyalkylsilane treated iron oxide pigments include without limitation Triethoxycaprylylsilane-treated iron oxide pigment. Perfluoroalkyl trialkyaxysilane-treated iron oxide pigments include without limitation Perfluorooctyl Triethoxysilane-treated iron oxide pigments. However, the choice of hydrophobically-modified iron oxide pigments is not particularly limited when employer in combinations with carbon black and the ratios of percent surface treatment to particle size discussed in reflation to other embodiment do not strictly apply to embodiments having preferably synergistic combinations with carbon black.

In another aspect of the invention or disclosure, cosmetic films are provided comprising a hydrophobic film-forming polymer and a pigment selected from the group consisting of alkylsilane-treated iron oxide, fluofosilane-treated iron oxide, fluoroalkylsilane-treated iron oxide, perfluaroalkylsilane-treated iron oxide, carbon black, and combinations thereof; the film being characterized by a contact angle with water of at least 140°, Also provided are substrates, such as keratin fibers (e.g., eyelashes), having disposed thereon a cosmetic film according to the invention.

Methods for imparting a hydrophobic film to the eyelashes are provided comprising applying thereto a composition according to the invention and allowing the volatile constituents to evaporate, thereby forming a superhydrophobic film characterized by a contact angle with a water droplet of at least 140°.

The one or more hydrophobic film formers for inclusion in the compositions of the invention are not particularly restricted and may comprise, for example, a film former selected from the group consisting of (alkyl)acrylates, polyurethanes, fluoropolymers, silicones, and copolymers thereof. A preferred hydrophobic film formers is an acrylates/dimethicone copolymer. The one or more hydrophobic film formers may also comprise a copolymer of two or more blocks selected from styrene (S), alkylstyrene (AS), ethylene/butylene (EB), ethylene/propylene (EP), butadiene (B), isoprene (I), acrylate (A) and methacrylate (MA). A representative polymer according to this embodiment is Ethylene/Propylene/Styrene copolymer.

To achieve the desisted superhydrophobic effect, the aggregate weight percentage of all non-volatile water-soluble or water-dispersible organic constituents (i.e., non-volatile hydrophilic organic molecules) in the compositions should be less than 15%, typically below 10%, preferably below 5%, and ideally below 2%; and the weight percentage of all polyols, including the humectant glycerin, should be collectively below 5%, preferably below 2%, and ideally below 1% by weight, based on the entire weight of the composition; because such components tend to attract water and coat the surface of the film and consequently reduce the hydrophobicity thereof,

The compositions may be useful for a variety of products, including cosmetic products (mascara, foundation, eye shadow, lipstick, nail polish, etc.); skin care products; sunscreens,; hair care products; and pet care products, to name a few. In a preferred implementation, the compositions are formulated as mascara products and are capable of imparting long-wear, transfer-resistance, and water-repellency to the eyelashes.

Methods for providing a superhydrophobic film on the skin or hair are also provided. The methods generally comprise depositing on skin or hair a composition according to the invention and allowing the volatile constituents to evaporate, thereby forming a hydrophobic film characterized by a contact angle with a water droplet of at least 140°.

These and other aspects of the present invention will become apparent to those skilled in the art after a reading of the following detailed description of the invention, including the figures and appended claims.

BRIEF DESCRIPTION OF FIGURES

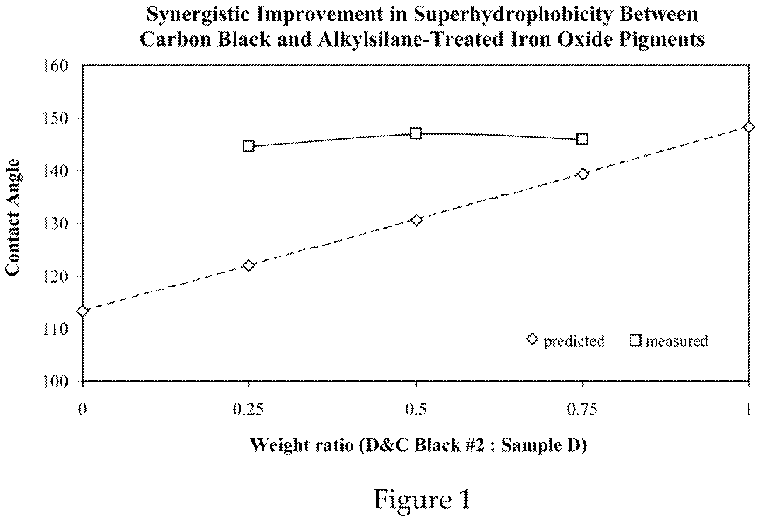

Figure 1 is a plot of contact angle as a function of the ratio of carbon black pigment to alkylsilane-treated iron oxide pigment, A synergetic improvement in superhydrophobicity is seen for the measured values of the combinations, indicated by the solid line and marker symbol (□), as compared to the predicted values based on the individual contributions of carbon black and alkylsilane-treated iron oxide pigments, as indicated by the dashed line and marker symbol (◇).

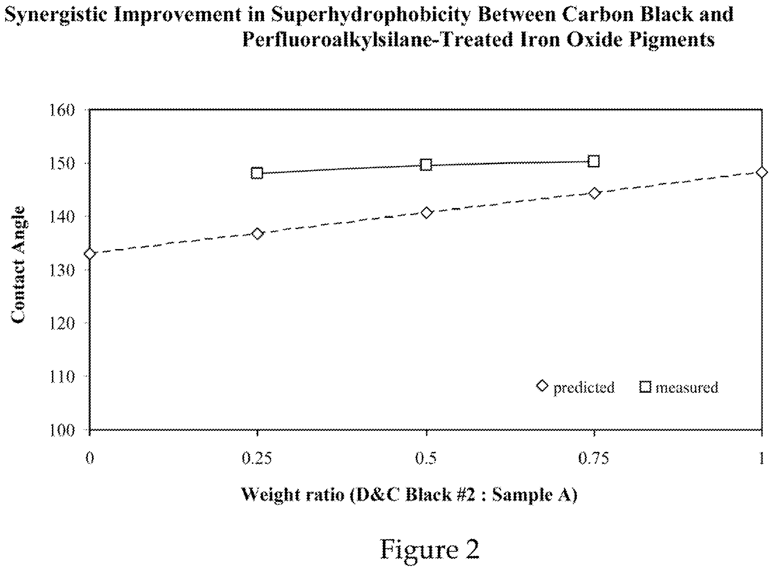

Figure 2 is a plot of contact angle as a function of the ratio of carbon black pigment to perfluoroalkylsilane-treated iron oxide pigment. A synergetic improvement in superhydrophobicity is seen for the measured values of the combinations, indicated by the solid line and marker symbol (□), as compared to the predicted values based on the individual contributions of carbon black and perfluoroalkylsilane-treated iron oxide pigments, as indicated by the dashed line and marker symbol (◇).

DETAILED DESCRIPTION

As used herein, the term "superhydrophobic" refers generally to any surface which gives a contact angle with water of greater than about 140°. Superhydrophobicity can be quantitatively evaluated by measuring the contact angle with water using a contact angle goniometer or other like method known in the art or may be qualitatively evaluated by visual inspection and observation of water repellency, i.e., observation of water beads rolling off a cast film.

All references to median or mean particle sizes herein are on a volume basis. All amounts provided in terms of weight percentage are relative to the entire composition unless otherwise stated. Unless otherwise provided, the term "alkyl" is intended to embrace straight-chained, branched, or cyclic hydrocarbons, particularly those having from one to 20 carbon atoms, and more particularly $C_{1-12}$ hydrocarbons,

Superhydrophobicity provides water repellency to a surface and consequently will affect the long-wear properties and self-cleaning properties of cosmetic compositions following administration to the skin, nails, or hair. In addition, it is thought that compositions according to the invention reduce adhesivity of pollutants, dirt, and the like to skin, nails, or hair because of a mismatch in surface energy. As a result, pollutants, dirt, and the like are more easily removed with or without water, resulting in self-cleaning properties, More importantly, the compositions provide a barrier against water such that the skin or hair does not become wet or is only poorly wettable on contact with water, e.g. sweat, rain, etc,

The inventive cosmetic compositions for imparting superhydrophobic films may advantageously be in the form of a mascara, and will generally be anhydrous, although they may suitably be formulated as water-in-oils emulsions. As used herein, the water-in-oil emulsions include water-in-silicone emulsion.

The compositions are preferably capable of providing a film on a surface, after evaporation of volatile solvents, which is characterized by a contact angle with a water droplet greater than about 140°, preferably greater than about 145°, and more preferred still, greater than about 150°. The contact angle is a measure of the hydrophobicity of the surface and is the angle at which a liquid/vapor interface meets a solid surface. Contact angles are suitably measured using a contact angle goniometer, In various embodiments, the contact angle with water will be about 140°, about 141°, about 142°, about 143°, about 144°, about 145°, about 146°, about 147°, about 148°, about 149°, or about 150°.

The first required component of the composition according to the invention is a film-former. The film former preferably comprises a hydrophobic material. The hydrophobic film former may be any hydrophobic film former suitable for use in a cosmetic composition including, but not limited to, hydrophobic film-forming polymers, The term film-forming polymer may be understood to indicate a polymer which is capable, by itself or in the presence of at least one auxiliary film-forming agent, of forming a continuous film which adheres to a surface and functions as a binder for the particulate material. The term "hydrophobic" film-forming polymer will typically refer to a polymer with a solubility in water at 25°C of less than about 1% by weight or one in which the monomeric units of the polymer individually have a solubility in water of less than about 1% by weight at 25°C, Alternatively, a "hydrophobic" film forming polymer may be said to be one which partitions preponderantly into the octanol phase when shaken with a mixture of equal volumes of water and octanol. By predominately is meant more the 50% by weight, but preferably more than 75% by weight, more preferably more than 95% by weight will partition into the octanol phase.

The film formers can be either natural or synthetic, polymeric or non polymeric, resins, binders, with low or high molar mass. Polymeric film formers can be either natural or synthetic, addition or condensation, homochain or heterochain, monodispersed or polydispersed, organic or inorganic, homopolyrners or copolymers, linear or branched or crosslinked, charged or uncharged, thermoplastic or thermoset, elastameric, crystalline or amorphous or both, isotactic or syndiotactic or atactic.

Polymeric film formers include polyolefins, polyvinyls, polyacrylates, polyurethanes, silicones, polyamides, polyesters, fluoropolymers, polyethers, polyacetates, polycarbonates, polyimides, rubbers, epoxies, formaldehyde resins, and homopolymers and copolymers of and of the foregoing.

Suitable hydrophobic (lipophilic) film-forming polymers include, without limitation, those described in U.S. Patent Nos. 7,037,515 to Kalafsky, et al,; 6,685,952 to Ma et al.; 6,464,969 to De La Poterie, et al.; 6,264,933 to Bodelin, et al.; 6,683,125 to Keller et al.; and 5,911,980 to Samour, et al.

Copolymers comprising one or more blocks selected from styrene (S), alkylstyrene (AS), ethylene/butylene (EB), ethylene/propylene (EP), butadiene (B), isoprene (I), acrylate (A) and methacrylate (MA), or a combination thereof, are contemplated to be suitable hydrophobic film formers. Particular mention is made of Ethylene/Propylene/Styrene and Butylene/Ethylene/Styrene copolymer including those sold under the trade name Versagel MD 1600 from Penreco as Gellants in IDD,

Special mention may be made of polyalkylenes, and in particular $C_2$-$C_{20}$ alkene copolymers, such as polybutene; alkylcelluloses with a linear or branched, saturated or unsaturated $C_1$-$C_8$ alkyl radical, such as ethylcellulose and propylcellulose; copolymers of vinylpyrrolidone (VP) and in particular copolymers of vinylpyrrolidone and of $C_2$ to $C_{40}$ and better still $C_3$ to $C_{20}$ alkene, including the copolymers of vinyl pyrollidone with eicosene or dodecane monomers sold under the tradenames Ganex V 220 and Ganex V 216 polymers (ISP Inc. of Wayne, NJ); silicones polymers and polyorganosiloxanes, including without limitations, polyalkyl siloxane, polyaryl siloxane, or a polyalkylaryl siloxane, with special mention being made of polydimethylsiloxanes; polyanhydride resins such as those available from Chevron under the trade name PA-18; copolymers derived from maleic anhydride and $C_3$ to $C_{40}$ alkenes such as octadecene-1; polyurethane polymers, such as Performa V 825 (New Phase Technologies) and those disclosed in U.S. Patent No. 7,150,878 to Gonzalez, et al.; and polymers and copolymers made from esters of vinylic acid monomers, including without limitation (meth)acrylic acid esters (also referred to as (meth)acrylates), for example, alkyl (meth)acrylates, wherein the alkyl group is chosen from linear, branched and cyclic ($C_1$ -$C_{30}$) alkyls, such as, for example, ($C_1$-$C_{20}$) alkyl (meth)acrylates, and further still ($C_6$-$C_{10}$) alkyl (meth)acrylates. Among the alkyl (meth)acrylates which may be mentioned are those chosen from methyl methacrylate, ethyl methacrylate, butyl methacrylate, isobutyl methacrylate, 2-ethylhexyl methacrylate, lauryl methacrylate, and the like. Among the aryl (meth)acrylates which may be mentioned are those chosen from benzyl acrylates, phenyl acrylate, and the like, The alkyl group of the foregoing esters may be chosen, for example, from fluorinated and perfluorinated alkyl groups, that is to say that some or all of the hydrogen atoms of the alkyl group are replaced with fluorine atoms. Mention may also be made of amides of the acid monomers such as (meth)acrylamides, for example, N-alkyl(meth)acrylamides, such as ($C_1$-$C_{20}$) alkyls, including without limitation, N-ethylacrylamide, N-t-butylacrylamide, N-t-octylacrylamide and N-undecylacrylaznide. Vinyl polymers for the hydrophobic film-forming polymer may also result from the homopolymerization or copolymerization of at least one monomer chosen from vinyl esters, olefins (including fluoroolefins), vinyl ethers, and styrene monomers. For example, these monomers may be copolymerized with at least one of acid monomers, esters thereof, and amides thereof, such as those mentioned above. Non-limiting examples of vinyl esters which may be mentioned are chosen from vinyl acetate, vinyl neodecanoate, vinyl pivalate, vinyl benzoate and vinyl t-butylbenzoate. Among the olefins which may be mentioned are those chosen, for example, from ethylene, propylene, butene, isobutene, octene, octadecene, and polyfluorinated olefins chosen, for example, from tetrafluoroethylene, vinylidene fluoride, hexafluoropropene and chlorotrifluoroethylene. Styrene monomers which may be mentioned are chosen, for example, from styrene and alpha-methylstyrene. The list of monomers given is not limiting, and it is possible to use any monomer known to those skilled in the art which falls within the categories of acrylic and vinyl monomers (including monomers modified with a silicone chain) which result in hydrophobic films. In this regard, particular mention may be made of the commercially available film formers Cyclopentasiloxane (and) Acrylates/Dimethicone Copolmer (KP-545, Shinetsu Chemical Co., Ltd).

Other film formers known in the art can be used advantageously in the composition. These include acrylate copolymers, acrylates $C_{12-22}$ Alkyl methacrylate copolymer, acrylate/octylacrylamide copolymers, acrylate/VA copolymer, amodimethicone, AMP/acrylate copolymers, behenyl/isostearyl, butylated PVP, butyl ester of PVM/MA copolymers, calcium/sodium PVM/MA copolymers, dimethicone, dimethicone copolymers, dimethicone/mercaptopropyl methicone copolymer, dimethicone propylethylenediamine behenate, dimethicolnol ethylcellulose, ethylene/acrylic acid copolymer, ethylene/MA copolymer, ethylene/VA copolymer, fluoro $C_{2-8}$ alkyldimethicone, $C_{30-38}$ olefin/isopropyl maleate/MA copolymer, hydrogenated styrene/butadiene copolymer, hydroxyethyl ethylcellulose, isobutylene/MA copolymer, methyl methacrylate crosspolymer, methylacryloyl ethyl betaine/acrylates copolymer, octadecene/MA copolymers, octadecene/maleic anhydride copolymer, octylacrylamide/acrylate/butylaminoethyl methacrylate copolymer, oxidized polyethylene, perfluoropolymethylisopropyl ether, polyethylene, polymethyl methacrylate, polypropylene, PVM/MA decadiene crosspolymer, PVM/MA copolymer, PVP, PVP/decene copolymer, PVP/eicosene copolymer, PVP/hexadecene copolymer, PVP/MA copolymer, PVP/VA copolymer, sodium acrylate/vinyl alcohol copolymer, stearoxy dimethicone, stearoxytrimethylsilane, stearyl alcohol, stearylvinyl ether/MA copolymer, styrene/DVB copolymer, styrene/MA copolymer, tetramethyl tetraphenyl trisiloxane, tricontanyl PVP, trimethyl pentaphenyl trisiloxane, tximethylsiloxysilicate, VA/crotonates copolymer, VA/crotonates/vinyl proprionate copolymer, VA/butyl maleate/isobornyl acrylate copolymer, vinyl caprolactam/PVP/dimethylaminoethyl methacrylate copolymer, and vinyldimethicone.

Additional non-limiting representatives of hydrophobic film-forming polymers include at least one polycondensate chosen from polyurethane, polyurethane-acrylics, polyurethane-polyvinylpyrrolidones, polyester-polyurethanes, polyether-polyurethanes, polyureas and polyurea/polyurethanes. The polyurethanes may be for example, at least one chosen from aliphatic, cycloaliphatic, and aromatic polyurethane, palyurealurethanes, and polyurea copolymers comprising at least one of: at least one sequence of at least one aliphatic polyester origin, cycloaliphatic polyester origin, and aromatic polyester origin at least one branched and unbranched silicone sequence, for example, from polydimethylsiloxane and polymethylphenylsiloxane, and at least one sequence comprising fluorinated groups. Additional non-limiting representatives of poly condensates may be chosen from polyesters, polyesteramides, fatty-chain polyesters, polyamides resins, epoxyester resins, arylsulphonamide-epoxy resins, and resins resulting from the condensation of formaldehyde with an arylsulphonamide.

The hydrophobic film may also be formed *in situ* by employing a resin which cures after application to the skin, nails, or hair, including for example, a polydimethylsiloxane film formed by *in situ* hydrosilation of a hydrosilane and an olefinic-substituted siloxane or by *in situ* polycondensation of alkoxy-functionalized siloxanes.

Preferred polymeric film formers include acrylates, alkyl acrylates, polyurethane, fluoropolymers such as Fluomer (polyperfluoroperhydrophenanthrene) and silicone polymers. Particularly preferred are silicone acrylates such as acrylates/dimethicone copolymers sold under the trade names KP-545 or KP 550 (Shin-Etsu).

Other film formers that may be employed include, without limitation, natural, mineral and/or synthetic waxen. Natural waxes are those of animal origin, including without limitation beeswax, spermaceti, lanolin, and shellac wax, and those of vegetable origin, including without limitation carnauba, candelilla, bayberry, and sugarcane wax, and the like. Mineral waxes contemplated to be useful include, without limitation ozokerite, ceresin, montan, paraffin, microcrystalline, petroleum, and petrolatum waxes. Synthetic waxes include, for example, Fischer Tropsch (FT) waxes and polyolefin waxes, such as ethylene homopolymers, ethylene-propylene copolymers, and ethylene-hexene copolymers. Representative ethylene homopolymer waxes are commercially available under the tradename POLYWAX® Polyethylene (Baker Hughes Incorporated). Commercially available ethylene-α-olefin copolymer waxes include those sold under the tradename PETROLITE® Copolymers (Baker Hughes Incorporated). Another wax that is suitable is dimethiconol beeswax available from Noveon as ULTRABEE™ dimethiconol ester.

In some embodiments, it may be desirable to add a hydrophilic or water-soluble film former (e.g., cellulosics, polysaccharides, polyquaterniums, etc.) to the composition to improve spreading, emulsion stability, etc, While less preferred, it is within the scope of the invention to include such hydrophilic or water-soluble film formers. There is no restriction on the amount of hydrophilic or water-soluble film former, although at high levels (e.g., greater than 20% by weight based on the total weight of film former) it may be necessary to increase the ratio of hydrophobic particulate to film former to counter the reduction in surface hydrophobicity. In some embodiments, the collective weight percentage of hydrophilic or water-soluble film formers will be less than about 20%, preferably less than about 15%, more preferably less than about 10%, and more preferred still, less than about 5% by weight based on the total weight of all film formers. In a preferred embodiment, hydrophilic film formers will comprise less than about 2% by weight of the total weight of film formers in the emulsion. In one embodiment, the emulsion is substantially free of water-soluble film formers by which is meant that the amount of water-soluble film formers present does not impart a measurable difference in contact angle with water as compared to an otherwise identical composition in the absence of water-soluble film formers.

Combinations of any of the foregoing film formers are also contemplated to be suitable, including combinations or polymeric and non-polymeric film formers.

A second essential component according to the invention is a particulate material which is either hydrophobic by nature or has been hydrophobically modified by surface treatment or the like. As used herein, a particulate material which is hydrophobic by nature includes polymeric particulates comprising hydrophobic organic polymers (as defined above) as well as inorganic particulates, the surface of which is hydrophobic, including for example, elemental carbon-based particulates. As used herein, a hydrophobically-modified particle is one which is rendered less hydrophilic or more hydrophobic by surface modification as compared to the pigment in the absence of surface modification.

While not wishing to be bound by theory, it is thought that the particulate materials provide nano-scale (1 nm to -1,000 nm) or micro-scale (1 $\mu$m to ~200 $\mu$m) surface roughness or structure on the film, which imparts superhydrophobicity by providing protuberances on which water droplets may sit, thereby minimizing contact of the water with the surface at large, i.e., reducing surface adhesion. Surface roughness can be observed or measured by AFM, SEM, and the like. In some, but not all, embodiments, the particulate materials are not porous.

A preferred particulate material according to the invention is hydrophobically modified iron oxide. As used herein, the term "iron oxide" is intended to include, without limitation, the species $FeO$, $Fe_2O_3$, $Fe_3O_4$, and combinations thereof, and hydrates thereof, as well as all pigments having the INCI name Iron Oxides, such as Black Iron Oxide, Red Iron Oxide, Yellow Iron Oxide, Brown Iron Oxide, Orange Iron Oxide, Blue Iron Oxide, and the like. Such pigments are often designated in the art as Iron Oxides (CI 77489, CI 77491, CI 77492, CI 77499, etc.). Black Iron Oxides (CI 77499) are preferred.

The iron oxide pigments according to the invention are typically surface-treated to impart a hydrophobic coating, The surface treatment may be any such treatment that makes the particles more hydrophobic. The surface of the particles may, for example, be covalently or ionically bound to an organic molecule or silicon-based molecule or may be adsorbed thereto, or the particle may be physically coated with a layer of hydrophobic material. There is essentially no limitation on the nature of the hydrophobic treatment and alkyl, aryl, or allyl silanes, silicones, dimethicone, fatty acids (e.g., stearates), polymeric silanes may be mentioned as well as fluoro and perfluoro derivatives thereof, The hydrophobic compound may be attached to the iron oxide particle through any suitable coupling agent, linker group, or functional group (e.g., silane, ester, ether, etc), The hydrophobic compound comprises a hydrophobic portion which may be selected from, for example, alkyl, aryl, allyl, vinyl, alkyl-aryl, aryl-alkyl, organosilicone, and fluoro- or perfluoro-derivatives thereof. Hydrophobic polymeric coatings including polyurethanes, epoxys and the like, are also contemplated to be useful,

Hydrophobically modified particulates and methods for preparing hydrophobically modified particulates are well-known in the art, as described in, for example, U.S. Patent No, 3,393,155 to Schutte et al., U.S. Patent No. 2,705,206 to Wagner et al., U.S. Patent No. 5,500,216 to Wagner et al., U.S. Patent No, 6,683,126 to Keller et al., and U.S. Patent No, z8 to Müller et al., Ups. Patent Pub, No. 2006/0110541 to Russell at al., and U.S. Patent Pub. No. 2006/0110542 to Dietz et al. In one embodiment, a hydrophobic particle in accordance with an embodiment of the present invention may be formed from an iron oxide particle having its surface covered with (e.g., covalently bonded to) non-polar radicals, such as for example alkyl groups, silicones, siloxanes, alkylsiloxanes, organosiloxanes, fluorinated siloxanes, perfluorosiloxanes, organosilanes, alkylsilane, fluorinated silanes, perfluorinated silanes and/or disilazanes and the like. U.S. Patent No. 6,315,990 to Farer, et al. describes fluorosilane coated particulates which are formed by reacting a particulate having nucleophilic groups, such as oxygen or hydroxyl, with a silican-oorltaining compound having a hydrocarbyl group substituted by at least one fluorine atom and a reactive hydrocarbyloxy group capable of displacement by a nucleophile. An example of such a compound is tridecafluorooctyltriethoxy silane, available from Sivento, Piscataway, N.J., under the trade name DYNASILANE™ F 8261.

In one embodiment, the iron oxide pigment has been surface treated with an alkylsilane, such as a $C_{1-20}$ alkylsilane, or more typically a $C_{1-12}$ alkylsilane, including an exemplary embodiment wherein the iron oxide is surface-treated with a $C_8$ alkylsilane (e.g., caprylylsilane). The pigments may be prepared by treating iron oxide with a trialkoxyalkylsilane, such as Triethoxycaprylylsilane (INCI), Iron oxide pigments surface-functionalized with caprylylsilane groups are available under the trade names AS-5146 Alkyl Silane Treated Black Oxide (Color Techniques), As-5123 Alkyl Silane Treated Red Oxide (Color Techniques), AS-5126 Alkyl Silane Treated Red Oxide (Color Techniques), AS-5131 Alkyl Silane Treated Yellow Oxide (Color Techniques), AS-5137 Alkyl Silane Treated Yellow Oxide (Color Techniques), Black Iron Oxide AS (Cardre), Red Iron Oxide AS (Cardre), Yellow Iron Oxide AS (Cardre), and Black NF-11S2 (Kobo), to name a few.

In another embodiment, the iron oxide pigment has been surface treated with a fluoroalkylsilane, and in particular a perfluoroalkylsilane, such as a $C_{1-20}$ perfluoroalkylsilane, or more typically a $C_{1-12}$ perfluoroalkylsilane, including an exemplary embodiment wherein the iron oxide is surface-treated with a $C_8$ perfluoroalkylsilane. The pigments may be prepared by treating iron oxide with a trialkoxyfluoroalkylsilane, such as Perfluorooctyl Triethoxysilane (INCI). Suitable iron oxide pigments surface-functionalized with perfluorooctylsilane groups are available under the trade names Cardre Black Iron Oxide FS (Cardre), Cardre Red Iron Oxide FS (Cardre), Cardre Yellow Iron Oxide FS (Cardre), and under the Unipure line from Sensient, including Unipure Black LC 989, to name a few.

The iron oxide pigments will typically, though not necessarily, have a mean (average) particle size between about 0,05 $\mu$m and about 20 $\mu$m, more typically, between about 0.1 $\mu$m and about 15 $\mu$m. In various embodiments, the iron oxide pigments will have a mean particle size between about 0.1 $\mu$m and about 5 $\mu$m, between about 0.2 $\mu$m and about 2.5 $\mu$m, or between about 0.25 $\mu$m and about 2.5 $\mu$m. In certain non-limiting embodiment, the iron oxide pigments will have a particle size between about 0.4 and about 0.75 and about 1.75 $\mu$m.

The degree of surface treatment of commercially available iron oxide pigments varies substantially. As used herein, the extent of surface treatment is expressed as percent surface treatment (ST) and is calculated as the weight ratio of the surface treatment agent (e.g., triethoxyalkylsilane, perflouroalkyl triethoxysilane, etc.) to the metal oxide component (e.g., iron oxide) expressed as a percentage, Thus, in the exemplary case of a TriethoxycaprylylSilane (INCI) treated Black Iron Oxide, such as Black NF 1152 (Kobo) or Black Iron Oxide AS (Cadre), the percent surface treatment (ST) is given as follows:

$$ ST = 100 \text{ x} \left( \frac{W_1}{W_2 + W_1} \right) $$

where $W_1$ is the weight of Triethoxycaprylylsilane and $W_2$ is the weight of metal oxide, in this case iron oxide, The degree of surface treatment ST will typically range from about 0.5% to about 5%, though it is more common for commercial iron oxide to have ST values in the range of about 0.8% to about 3%.

It has been found that many iron oxide pigments are not capable of producing superhyrophobic films, in part because the percent surface treatment is low, However, low ST, alone, is not necessarily detrimental to the effect provided that the mean particle size of the pigment is also very small such that the alkyl chains interact with a large surface area of the particle. As the mean particle size of the pigment is increased, it is theorized that the percent surface treatment (ST) must also be increased (for a given alkyl or perfluoroalkyl chain length) in order to achieve a superhydrophobic film because increased ST is required to maintain the hydrophobic character of the particle. Thus, it has been found that the parameter defined by the ratio of ST to the mean particle size in microns ("mean") is highly correlative with the hydrophobicity of a film, This parameter is referred to as "ST:mean" herein.

The precise value of ST:mean required to achieve a superhyrophobic film will depend on the nature of the surface treatment (perfluoroalkyl chains provide more hydrophobicity than corresponding alkyl chains). In general, it may be said that, ST:mean ratios of 0,5, 0.75, 1, 1.1, 1.2, 1.3, 1.4, 1.5, 1.6, 1.7, 1.8, 1.9, 2.0, 2.1, 2,2, 2.3, 2.4, 2.5, 2.6, 2.7, 2.8, 2.9, 3.0, 3.25, 3.5, 3.75, 4, 4.25, 4.5, 5.75, 6, 7, 8, 9, and 10 are each considered to be a distinct embodiment of the invention

In the case of surface treatments comprise one or more fluorine atoms, such as fluoroalkylsilane and perfluoroalkylsilane surface treatments, ST:mean values greater than 1.1 are desirable. More typically, the ST:mean value will be greater than 1.2, greater than 1.3, greater than 1,4, greater than 1.5, greater than 1.6, greater than 1.7, greater than 1.8, greater than 1.9, or greater than 2.0,

Similarly, in the case of alkyl, aryl, or silicone-based surface treatments that do not comprise fluorine atoms, including alkyl silanes, silicone, dimethicone, stearates, polymeric silanes surface treatments or the like, ST;mean values greater than about 2.3 are desirable, More typically, the ST:mean value will be greater than 2.4, greater than 2.5, greater than 2.6, greater than 2,7, greater than 2,8, greater than 2.9, or greater than 3,0,

It will be observed that the ST:mean value required to achieve a superhydrophobic surface where the iron oxide is surface-functionalized with alkyl groups is roughly twice that required where the iron oxide is surface-functionalized with the corresponding perfluoroalkyl groups,

The particulate material according to the invention is carbon black, Suitable carbon black powders will typically have a mean particle size of about 0.01 $\mu$m to about 5 $\mu$m, more typically between about 0.01 and about 1 $\mu$m, and preferably between about 0.01 and about 0.1 $\mu$m (i.e., about 10 to about 100 nanometers). The carbon black powder may have a surface area between about 50 and about 500 meters $(m)^2$/gram, more typically between about 100 and about 350 $m^2$/gram, and more typically between about 150 and about 300 $m^2$/gram as measured by nitrogen BET, A suitable carbon black is D&C Black No. 2 which is formed by the combustion of aromatic petroleum oil feedstock and consists essentially of pure carbon, formed as aggregated fine particles with a surface area range of 200 to 260 meters $(m)^2$/gram by nitrogen BET, D&C Black No. 2 is available from Sensient under the tradename Unipure black LC 902.

This material has a mean particle size of about 0.04 $\mu$m.

It is contemplated that synergistic improvements in hydrophobicity will be obtained by using combinations of hydrophobically-modified iron oxide pigments and carbon black pigments. The combinations are expected to be synergistic over all ratios of iron oxide pigment to carbon black, although in certain embodiments the ratio of iron oxide pigment to carbon black will range from about 1:10 to about 10:1, more typically from about 1:5 to about 5:1, including ranges of about 1:4 to about 4:1, about 1:3 to about 3:1, about 1:2 to about 2:1, and equal weight mixtures having a ratio of about 1:1. The synergistic combinations will provide films having a contact angle with water great than what would be expected were the contribution of each component merely additive.

The ratios of the particulates to the film forgers in the compositions according to the invention are controlled to produce compositions with the desired superhydrophobic effect. The iron oxide and/or carbon black pigments will typically be present in the aggregate, in both the compositions and films, at a weight ratio to the hydrophobic film formers of about 1:10 to about 10:1 or from about 1:5 to about 5:1. For example, the weight of hydrophobic film-former to particulate material may range from about 1:2 to about 2:1, including the ratio of about 1:2, about 1:1.75, about 1:1.5, about 1:1.25, about 1:1, about 1,25:1, about 1.5:1, about 1,75:1, and about 2:1. Particularly good results have been obtained where the weight ratio of hydrophobic film-former to particulate material is about 1;1.

In addition to the hydrophobically modified iron oxide and/or carbon black pigments, the compositions may further comprise one or more additional hydrophobic particulate materials. A preferred particulate material according to the invention or disclosure is hydrophobically modified silica ($SiO_2$) powder, including fumed silica or pyrogenic silica (e.g., having a particle size range from about 7 nm to about 40 nm). Other notable particulate materials are hydrophobically modified metal oxides and metalloid oxides, including without limitation titanium dioxide ($TiO_2$), aluminum oxide ($Al_2O_3$), zirconium dioxide ($ZrO_2$), tin dioxide ($SnO_2$), zinc oxide (ZnO), and combinations thereof.

Advantageously, the particulate material may be one which provides additional functionality to the compositions, including for example, ultraviolet (UV) light absorption or scattering, in the case of, for example, titanium dioxide and zinc oxide particulates, or provide aesthetic characteristics, such as color (e.g., pigments), pearlesence (e.g. mica), or the like. The particulate material may be based, for example, on organic or inorganic particulate pigments. Examples of organic particulate pigments include lakes, especially aluminum lakes, strontium lakes, barium lakes, and the like. Examples of the inorganic particulate pigments are iron oxide, especially red, yellow and black iron oxides, titanium dioxide, zinc oxide, potassium ferricyanide ($K_3Fe(CN)_6$), potassium ferrocyanide ($K_4Fe(CN)_6$), potassium ferrocyanide trihydrate ($K_4Fe(CN)_6 \cdot 3H_2O$), and mixtures thereof. The particulate material may also be based on inorganic fillers such as talc, mica, silica, and mixtures thereof, or any of the clays disclosed in EP 1 640 419.

Any of the hydrophobically modified particulate materials described in U.S. Patent No. 6,683,126 to Keller et al. are also contemplated to be useful, including without limitation those obtained by treating an oxide material (e.g., $SiO_2$, $TiO_2$, etc.) with a (perfluoro)alkyl-containing compound that contains at least one reactive functional group that undergoes a chemical reaction with the near-surface -OH groups of the oxide support particle, including for example hexamethyldisilazane, octyltrimethoxysilane, silicone oil, chlorotrimethylsilane, and dichlorodimethylsilane.

Suitable hydrophobically modified fumed silica particles include, but are not limited to AEROSIL™ R 202, AEROSIL™ R 805, AEROSIL™ R 812, AEROSIL™ R 812 S, AEROSIL™ R 972, AEROSIL™ R 974, AEROSIL™ R 8200, AEROXIDE™ LE-1, AEROXIDE™ LE-2, and AEROXIDE™ LE-3 from Degussa Corporation of Parsippany, N.J. Other suitable particulates include the particulate silicon wax sold under the trade name Tegotop™ 105 (Degussa/Goldschmidt Chemical Corporation) and the particulate vinyl polymer sold under the name Mincor™ 300 (BASF). While silica ($SiO_2$) and hydrophobically-modified silicas are contemplated to be particularly useful in some embodiments, in other embodiments the compositions will be substantially free of silica or hydrophobically-modified silica. By substantially free of silica or hydrophobically-modified silica means that these components comprise less than about 2%, preferably less than about 1%, and more preferably less than about 0.5% by weight of the one or more particulate materials. A suitable hydrophobically modified alumina particulate is ALU C 805 from Degussa. The hydrophobically modified silica materials described in U.S. Patent Pub. 2006/0110542 to Dietz et al. are contemplated to be particularly suitable. In some embodiments, the compositions will be substantially free of alumina or hydrophobically modified alumina.

The one or more particulate materials may also comprise particulate organic polymers such as polytetrafluoroethylene, polyethylene, polypropylene, nylon, poly-vinyl, chloride, polymethylmethacrylate (PMMA), cellulosics and the like which have been formed into fine powders. Alternatively, the particulate material may be a microcapsule comprising any of the shell materials described in U.S. Patent Pub. 2005/0000531.

The one or more additional particulate materials will typically be in the form of a powder having a median particle size between about 1 nm (nanometers) and about 1 mm (millimeters), more typically between about 5 nm and about 500 $\mu$m (micrometer), preferably between about 7 nm and about 1 $\mu$m, 5 $\mu$m, 20 $\mu$m, 50 $\mu$m or about 100 $\mu$m. Where more than one particulate material is employed (e.g., modified $TiO_2$ and modified $SiO_2$), the median particle size of each powder is preferably within the foregoing ranges.

Particulate materials having median particle sizes above about 1 mm may be too large, unless the particle itself contains surface roughness in the appropriate size range. For example, surface treatment of a larger particle with a polymer chain in the 20 nm range may provide acceptable surface roughness. Roughness of the resulting films may be characterized by the size of the primary particle, by the size of agglomerated particles in the aggregate, or by the distribution of particle sizes.

Generally, the weight ratio of the one or more hydrophobic film formers to the hydrophobic particulates will be from about 1:10 to about 10:1, about 1:10 to about 5:1, about 1:5 to about 5:1, about 1:5 to about 2:1, or about 1:2 to about 1:1, with higher levels of particulate material being preferred. Mention may be made of the following ratios of one or more hydrophobic film formers to the hydrophobic particulates: about 1:15, about 1:10, about 1:9, about 1:8, about 1:7, about 1:6, about 1:5, about 1:4, about 1:3, about 1:2, about 1:1.5, about 1:1, about 1.5:1, about 2:1, about 3:1, about 4:1, and about 5:1.

The one or more hydrophobic film formers and hydrophobic particulate materials will collectively comprise, for example, at least about 0.1%, 0.5%, 1%, 2%, 5%, 10%, 15%, 20%, or 25% by weight of the cosmetic composition up to about 30%, 40%, 50%, 60%, 70%, 80%, 90% or 100% of the total weight of the composition. In various embodiments, the one or more hydrophobic film formers and hydrophobic particulate materials will collectively comprise from about 0.1% to about 90%, or about 10% to about 75%, or about 25% to about 60% by weight of the total composition. In various embodiments, the one or more hydrophobic film formers and hydrophobic particulate materials will collectively comprise from about 1-5%, about 5-10%, about 10-15%, about 15-20%, about 20-25%, about 25-30%, about 30-35%, about 35-40%, about 40-45%, about 45-50%, about 50-55%, about 55-60%, about 60-65%, about 65-70%, about 70-75%, about 75-80%, about 80-85%, about 85-90%, about 90-95%, or about 95-100% based on the total weight of the composition.

Where the composition is formulated as a mascara, the collective weight of the one or more hydrophobic film formers and hydrophobic particulate materials will typically be between about 30% and about 70% by weight. In a hair product, which will typically comprise more volatiles than other cosmetic products, the collective weight of the one or more hydrophobic film formers and hydrophobic particulate materials will typically be between about 1% and about 25% by weight. In a liquid foundation, the collective weight of the one or more hydrophobic film formers and hydrophobic particulate materials will typically be between about 0.5% and about 30% by weight. In a powdered cosmetic, such as a rouge, the collective weight of the one or more hydrophobic film formers and hydrophobic particulate materials will typically be between about 1% and about 30% by weight. For a lipstick, the collective weight of the one or more hydrophobic film formers and hydrophobic particulate materials will typically be between about 30% and about 70% by weight.

The hydrophobic film-former and iron oxide pigments and/or carbon black particulate material may collectively comprise at least about 50%, at least about 60%, at least about 70%, or at least about 80% by weight of the non-volatile portion of the composition. Typically, the hydrophobic film-former and particulate material will collectively comprise less than about 95%, less than about 90%, or less than about 85% by weight of the non-volatile portion of the composition to accommodate other ingredients conventionally found in cosmetic products. In one embodiment the hydrophobic film-former and pigment materials collectively comprise about 80% to about 90% by weight of the non-volatile portion of the composition.

Anhydrous Formulations

The compositions of the invention may be provided as anhydrous formulations. By "anhydrous" is mean that the weight percentage of water in the composition is less than about 1% by weight. Preferably, the anhydrous compositions are substantially free of water by which is meant that water is not deliberately added to the compositions and the level of water is no more than would be expected based on the absorption of water from the air.

The anhydrous composition will typically comprise a volatile hydrophobic solvent, such as volatile hydrocarbons, volatile silicones, and the like. Among the volatile hydrocarbons, special mention may be made of isododecane which is available under the trade name Permethyl-99A (Presperse Inc.).

In the case where the iron oxide pigment is surface-treated with perfluoroalkyl groups, it is contemplated that additional advantages will be obtained by incorporating at least a small amount of a fluorinated solvents or polymers in the composition. It is believed that the use of fluorinated solvents or binders will increase the dispersibility of the pigments having perfluoroalkyl groups in the hydrophobic film formers and thus provide superior hydrophobicity of the resultant film. Suitable fluorinated solvents and polymers include, without limitation, perfluoroethers, perfluorodecalin, perfluoromethyldecalin, perfluorodimethylcyclohexane, perfluorohexane, perfluoroheptane, perfluorononane, perfluoromethylcyclohexane, perfluoromethylcycopentane, and fluorinated silicones, such as perfluorononyl dimethicone, for example. Such fluorintated solvents and polymers may be present in the composition in any amount, but typically will comprise from about 0.05% to about 20% by weight, more typically from about 0.1% to about 10% by weight, and preferably from about 0.5% to about 5% by weight.

Water-in-Oil Emulsion

The compositions according to the invention may be formulated as water-in-oil emulsions. These emulsions comprise an oil-containing continuous phase and an aqueous discontinuous phase

The oil-containing phase will typically comprise from about 10% to about 99%, preferably from about 20% to about 85%, and more preferably from about 30% to about 70% by weight, based on the total weight of the emulsion, and the aqueous phase will typically comprise from about 1% to about 90%, preferably from about 5% to about 70%, and more preferably from about 20% to about 60% by weight of the total emulsion. The aqueous phase will typically comprise from about 25% to about 100%, more typically from about 50% to about 95% by weight water.

The oil-containing phase may be composed of a singular oil or mixtures of different oils. Essentially any oil is contemplated to be useful, although highly hydrophobic oils are preferred. Suitable non-limiting examples include vegetable oils; esters such as octyl palmitate, isopropyl myristate and isopropyl palmitate; ethers such as dicapryl ether; fatty alcohols such as cetyl alcohol, stearyl alcohol and behenyl alcohol; isoparaffins such as isooctane, isododecane and isohexadecane; silicone oils such as dimethicones, cyclic silicones, and polysiloxanes; hydrocarbon oils such as mineral oil, petrolatum, isoeicosane and polyisobutene; natural or synthetic waxes; and the like.

Suitable hydrophobic hydrocarbon oils may be saturated or unsaturated, have an aliphatic character and be straight or branched chained or contain alicyclic or aromatic rings. Hydrocarbon oils include those having 6-20 carbon atoms, more preferably 10-16 carbon atoms. Representative hydrocarbons include decane, dodecane, tetradecane, tridecane, and $C_{8}$-$_{20}$ isoparaffins. Paraffinic hydrocarbons are available from Exxon under the ISOPARS trademark, and from the Permethyl Corporation. In addition, $C_{8}$-$_{20}$ paraffinic hydrocarbons such as $C_{12}$ isoparaffin (isododecane) manufactured by the Permethyl Corporation having the tradename Permethyl 99A™ are also contemplated to be suitable. Various commercially available $C_{16}$ isoparaffins, such as isohexadecane (having the tradename Permethyl R™) are also suitable. Examples of preferred volatile hydrocarbons include polydecanes such as isododecane and isodecane, including for example, Permethyl-99A (Presperse Inc.) and the $C_{7}$-$C_{8}$ through $C_{12}$-$C_{15}$ isoparaffins such as the Isopar Series available from Exxon Chemicals. A representative hydrocarbon solvent is isododecane.

What is critical is that the emulsions have little or no non-volatile hydrophilic constituents, including some conventional humectants. Components such as glycerin and polyols, including propylene glycol, ethoxydiglycol, glycerin, butylene glycol, pentylene glycol and hexylene glycol should be eliminated or should be kept at levels such that the non-volatile hydrophilic constituents, in the aggregate, do not exceed 15% by weight and preferably will be less than 10%, less than 5%, less than 2%, or less than 1% by weight. Glycerin has been found to be particularly detrimental to achieving superhydrophobicity and should therefore be maintained at levels below 2% by weight, or eliminated altogether.

It has been found that the selection and amount of emulsifier is important for obtaining films which provide superior hydrophobic properties. Because the emulsifier itself may be deleterious to the formation of a superhydrophobic film, the compositions preferably have the lowest level of emulsifier capable of producing a stable emulsion. The amount of emulsifier will typically be from about 0.001 wt % to about 10 wt %, but preferably will range from about 0.01 to about 5 wt %, and most preferably about 0.1 wt % to about 1 wt %, based upon the total weight of the composition.

For water in oil emulsions, the emulsifier itself should be of low HLB, preferably below 10, more preferably below 8.5. While combinations of more than one emulsifier are contemplated to be within the scope of the invention, each such emulsifier, individually, should be of low HLB. Therefore, the use of high and low HLB emulsifiers, which in combination give low HLB (e.g., less than 8.5), is less desirable because even if the combined HLB of the system is below 8.5, the contribution of the higher HLB emulsifier will be detrimental to the formation of a superhydrophobic film. If present, the amount of emulsifier having an HLB above 10 will be less than 1% by weight, more preferably less than 0.5% by weight, and more preferred still, lees than 0.2% by weight.

Where the emulsifier is of the polyethoxylated type (e.g., polyoxyethylene ethers or esters) comprising chains of the form -$(CH_2CH_2O)_n$-, it is preferred that n be less than 20, more preferably less than 10, most preferably less than 5. Propoxylated emulsifiers are also contemplated to be suitable. Propoxylated emulsifiers also preferably having less than 20, more preferably less than 10, most preferably less than 5 propylene oxide repeat units.

Emulsifiers that can be used in the composition of the present invention include, but are not limited to, one or more of the following: sorbitan esters; polyglyceryl-3-diisostearate; sorbitan monostearate, sorbitan tristearate, sorbitan sesquioleate, sorbitan monooleate; glycerol esters such as glycerol monostearate and glycerol monooleate; polyoxyethylene phenols such as polyoxyethylene octyl phenol and polyoxyethylene nonyl phenol; polyoxyethylene ethers such as polyoxyethylene cetyl ether and polyoxyethylene stearyl ether; polyoxyethylene glycol esters; polyoxyethylene sorbitan esters; dimethicone copolyols; polyglyceryl esters such as polyglyceryl-3-diisostearate; glyceryl laurate; Steareth-2, Steareth-10, and Steareth-20, to name a few. Additional emulsifiers are provided in the INCI Ingredient Dictionary and Handbook 11th Edition 2006.

An example of a very low HLB emulsifier contemplated to be suitable according to the invention is Span 83, a sesquiester of monooleate and dioleate at a 2:1 molar ratio which has an HLB of 3.7. Sorbitan monostearate (INCI) is another suitable emulsifier, having an HLB value of 4.7.

The aqueous phase may include one or more additional solvents, preferably volatile solvents, including lower alcohols, such as ethanol, isopropanol, and the like. The volatile solvent may also be a cosmetically acceptable ester such as butyl acetate or ethyl acetate; ketones such as acetone or ethyl methyl ketone; or the like. The volatile solvents, when present in the aqueous phase, will typically comprise from about 0.1% to about 75% by weight of the aqueous phase, more typically up to about 35% by weight, and preferably up to about 15% by weight. The water and optional volatile solvents are contemplated to enhance the formation of a superhydrophobic film because the particulates will tend to be pushed to the surface of the film as the solvents evaporate.

Water-in-Silicone Emulsion

One type of water-in-oil emulsion that has been found to be useful is a water-in-silicone emulsions having a silicone oil-containing continuous phase and an aqueous discontinuous phase.

The silicone-containing phase will typically comprise from about 20% to about 95%, preferably from about 25% to about 85%, and more preferably from about 35% to about 70 the aqueous phase will typically comprise from about 5% to about 90%, preferably from about 10% to about 70%, and more preferably from about 20% to about 60% by weight of the total emulsion. The aqueous phase will typically comprise from about 25% to about 100%, more typically from about 50% to about 95% by weight water.

The silicone oil phase may include volatile silicone oils, non-volatile silicone oils, and combinations thereof. By volatile silicone oil is meant that the oil readily evaporates at ambient temperatures. Typically, volatile silicone oils will exhibit a vapor pressure ranging from about 1 Pa to about 2 kPa at 25°C; will preferably have a viscosity of from about 0.1 to about 10 centistokes, preferably about 5 centistokes or less, more preferably about 2 centistokes or less, at 25°C; and will boil at atmospheric pressure at from about 35°C to about 250°C.

Volatile silicones include cyclic and linear volatile dimethylsiloxane silicones. In one embodiment, the volatile silicones may include cyclodimethicones, including tetramer (D4), pentamer (D5), and hexamer (D6) cyclomethicones, or mixtures thereof. Particular mention may be made of the volatile cyclomethicone-hexamethyl cyclotrisiloxane, octamethyl-cyclotetrasiloxane, and decamethyl-cyclopentasiloxane. Suitable dimethicones are available from Dow Corning under the name Dow Corning 200® Fluid and have viscosities ranging from 0.65 to 600,000 centistokes or higher. Suitable non-polar, volatile liquid silicone oils are disclosed in U.S. Pat. No. 4,781,917. Additional volatile silicones materials are described in Todd et al., "Volatile Silicone Fluids for Cosmetics", Cosmetics and Toiletries, 91:27-32 (1976). Linear volatile silicones generally have a viscosity of Less than about 5 centistokes at 25°C., whereas the cyclic silicones have viscosities of less than about 10 centistokes at 25°C. Examples of volatile silicones of varying viscosities include Dow Corning 200, Dow Coning 244, Dow Corning 245, Dow Corning 344, and Dow Corning 345, (Dow Corning Corp.); SF-1204 and SF-1202 Silicone Fluids (G.E. Silicones), GE 7207 and 7158 (General Electric Co.); and SWS-03314 (SWS Silicones Corp.). Linear, volatile silicones include low molecular weight polydimethylsiloxane compounds such as hexamethyldisiloxane, octamethyltrisiloxane, decamethyltetrasiloxane, and dodecamethylpentasiloxane to name a few.

Non-volatile silicone oils will typically comprise polyalkylsiloxanes, polyarylsiloxanes, polyalkylarylsiloxanes, or mixtures thereof. Polydimethylsiloxanes are preferred non-volatile silicone oils. The non-volatile silicone oils will typically have a viscosity from about 10 to about 60,000 centistokes at 25°C, preferably between about 10 and about 10,000 centistokes, and more preferred still between about 10 and about 500 centistokes; and a boiling point greater than 250°C at atmospheric pressure. Non limiting examples include dimethyl polysiloxane (dimethicone), phenyl trimethicone, and diphenyldimethicone.

The volatile and non-volatile silicone oils may optionally be substituted will various functional groups such as alkyl, aryl, amine groups, vinyl, hydroxyl, haloalkyl groups, alkylaryl groups, and acrylate groups, to name a few.

The water-in-silicone emulsion is emulsified with a nonionic surfactant (emulsifier). Suitable emulsifiers include polydiorganosiloxane-polyoxyalkylene block copolymers, including those described in U.S. Patent No.4, 122,029. These emulsifiers generally comprise a polydiorganosiloxane backbone, typically polydimethylsiloxane, having side chains comprising $-(EO)_m-$ and/or $-(PO)_n-$ groups, where EO is ethyleneoxy and PO is 1,2-propyleneoxy, the side chains being typically capped or terminated with hydrogen or lower alkyl groups (e.g., $C_{1-6}$, typically $C_{1-3}$). The side chains will preferably comprise 50 EO and/or PO units or less (e.g., m + n =<50), preferably 20 or less, and more preferably 10 or less. In addition to the alkoxylated side chain, the silicone emulsifier may also comprise alkyl chains pendant from the silicone backbone. Other suitable water-in-silicone emulsifiers are disclosed in U.S. Patent No. 6,685,952. Commercially available water-in-silicone emulsifiers include those available from Dow Corning under the trade designations 3225C and 5225C FORMULATION AID; SILICONE SF-1528 available from General Electric; ABIL EM 90 and EM 97, available from Goldschmidt Chemical Corporation (Hopewell, VA); and the SILWET™ series of emulsifiers sold by OSI Specialties (Danbury, CT).

Examples of water-in-silicone emulsifiers include, but are not limited to, dimethicone PEG 10/15 crosspolymer, dimethicone copolyol, cetyl dimethicone copolyol, PEG-15 lauryl dimethicone crosspolymer, laurylmethicone crosspolymer, cyclomethicone and dimethicone copolyol, dimethicone copolyol (and) caprylic/capric triglycerides, polyglyceryl-4 isostearate (and) cetyl dimethicone copolyol (and) hexyl laureate, and dimethicone copolyol (and) cyclopentasiloxane.

Preferred examples of water-in-silicone emulsifiers include, without limitation, PEG/PPG-18/18 dimethicone (trade name 5225C, Dow Coming), PEG/PPG-19/19 dimethicone (trade name BY25-337, Dow Corning), Cetyl PEG/PPG-10/1 dimethicone (trade name Abil EM-90, Goldschmidt Chemical Corporation), PEG-12 dimethicone (trade name SF 1288, General Electric), lauryl PEG/PPG-18/18 methicone (trade name 5200 FORMULATION AID, Dow Coming), PEG-12 dimethicone crosspolymer (trade name 9010 and 9011 silicone elastomer blend, Dow Coming), PEG-10 dimethicone crosspolymer (trade name KSG-20, Shin-Etsu), and dimethicone PEG-10/15 crosspolymer (trade name KSG-210, Shin-Etsu).

The water-in-silicone emulsifiers typically will be present in the composition in an amount from about 0.001% to about 10% by weight, in particular in an amount from about 0.01% to about 5% by weight, and more preferably, below 1% by weight.

In one embodiment of the invention, a composition for imparting a hydrophobic film on a surface comprises a water-in-oil emulsion. The water-in-oil emulsion includes (i) a continuous oil-phase; (ii) a discontinuous (internal) aqueous phase; (iii) an emulsifier having an HLB value less than 10, preferably less than 8.5; (iv) one or more hydrophobic film formers, and (v) hydrophobically-modified iron oxide pigments and/or carbon black.

In a related embodiment, a composition for imparting a hydrophobic film on a surface comprises a water-in-silicone emulsion. The water-in-silicone emulsion includes (i) a continuous silicone oil-phase; (ii) a discontinuous aqueous phase; (iii) an emulsifier comprising an organosiloxane polymer having side chains comprising $-(EO)_m-$ and/or $-(PO)_n-$ groups, where n and m are integers from zero to about 20 and where the sum of n and m is 50 or less, the side chains being terminated with hydrogen or lower alkyl groups; (iv) one or more hydrophobic film formers, and (v) hydrophobically-modified iron oxide pigments and/or carbon black.

In both the water-in-oil and water-in silicone emulsions, the weight ratio of the one or more hydrophobic film formers to the hydrophobically-modified iron oxide pigments and/or carbon black is as described above, and may suitably be, for example, from about 1:10 to about 10:1, about 1:10 to about 5:1, about 1:5 to about 5:1, about 1:5 to about 2:1, or about 1:2 to about 1:1, with higher levels of particulate material being preferred; and the one or more hydrophobic film formers and hydrophobically-modified iron oxide pigments and/or carbon black materials collectively comprise at least about 1% by weight, preferably at least about 2% by weight, more preferably at least about 5% by weight of the water-in-oil or water-in-silicone emulsion up to about 10%, 15%, 20%, 25%, 30%, 40%, 50%, 60%, 70%, 80%, or 90% of the total weight of the emulsion.

In the preferred practice of the invention, the hydrophobic film formers and hydrophobic pigments are first dispersed or dissolved in the oil or silicone phase. The oil or silicone is subsequently mixed with the aqueous phase to form an emulsion. The emulsions will typically have the hydrophobic film formers and hydrophobic pigments dispersed or dissolved predominantly in the oil or silicone phase.

Cosmetics

Cosmetic compositions according to the invention include, but are not limited to, color cosmetics, skin care products, hair care products, and personal care products. Color cosmetics include, for example, foundation and mascara. Skin care products include, but are not limited to, sunscreens, after-sun products, lotions, and creams. Additional applications include use in hair care products, insect repellents, deodorants, anti-perspirants, lipstick, ear canal product, baby wipes, baby creams or lotions, top coats to impart water-proofing or water-resistance to a previously applied cosmetic product, personal care product, hair care product, or first aid product. For example, the composition according to the invention could be applied as a top coat over a previously applied base coat to improve water-proofing or water-resistance. Similarly, the composition could be applied as a top coat over a first aid product such as an antibiotic ointment or spray, bandage, or wound dressing.

The preferred cosmetic according to the invention is a mascara. The compositions of the invention may further have any ingredient conventionally used in the cosmetic field, in particular in the manufacture of mascara products. The amounts of these various ingredients will typically range from about 0.01 to about 20 wt. % by weight of the composition. The nature of these ingredients and their amounts must be judiciously selected to not be deleterious to the superhydrophobic films. In this regard, it should be noted that the aggregate of all non-volatile, hydrophilic components should be kept below 15% by weight, and preferably below about 10% by weight of the composition.

In addition to the iron oxides pigments and/or carbon black, the mascara may comprise additional pigments, pearlescents, and/or colorants as is customary for such products. Inorganic pigments include titanium dioxide, zinc oxide, iron oxide, chromium oxide, ferric blue, and mica; organic pigments include barium, strontium, calcium or aluminium lakes, ultramarines, and carbon black; colorants include D&C Green #3, D&C Yellow #5, and D&C Blue #1. Pigments and/or colorants may be coated or surface treated with one or more compatibilizers to aid in dispersion in either or both of the aqueous or wax phases. Preferred pigments and/or colorants are those surface treated with dimethicone copolyol.

Various fillers and additional components may be added. Suitable fillers include without limitation silica, treated silica, talc, zinc stearate, mica, kaolin, Nylon powders such as Orgasol™, polyethylene powder, Teflon™, starch, boron nitride, copolymer microspheres such as Expancel™ (Nobel Industries), Polytrap™ (Dow Corning) and silicone resin microbeads (Tospearl™ from Toshiba), and the like.

Additional pigment/powder fillers include, but are not limited to, inorganic powders such as gums, chalk, Fuller's earth, kaolin, sericite, muscovite, phlogopite, synthetic mica, lepidolite, biotite, lithia mica, vermiculite, aluminum silicate, starch, smectite clays, alkyl and/or trialkyl aryl ammonium smectites, chemically modified magnesium aluminum silicate, organically modified montmorillonite clay, hydrated aluminum silicate, aluminum starch octenyl succinate barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, metallic soap (zinc stearate, magnesium stearate, zinc myristate, calcium palmitate, and aluminum stearate), colloidal silicone dioxide, and boron nitride; organic powder such as polyamide resin powder (nylon powder), cyclodextrin, methyl polymethacrylate powder, copolymer powder of styrene and acrylic acid, benzoguanamine resin powder, poly(ethylene tetrafluoride) powder, and carboxyvinyl polymer, cellulose powder such as hydroxyethyl cellulose and sodium carboxymethyl cellulose, ethylene glycol monostearate; inorganic white pigments such as magnesium oxide; and stabilizers/ rheology modifiers, for example, Bentone Gel and Rheopearl TT2. Other useful powders are disclosed in U.S. Pat. No. 5,688,831.

The mascara composition may comprise one or more waxes, including for example, rice bran wax, carnauba wax, ouricurry wax, candelilla wax, montan waxes, sugar cane waxes, ozokerite, polyethylene waxes, Fischer-Tropsch waxes, beeswax, microcrystaline wax, silicone waxes, fluorinated waxes, and any combination thereof.

The compositions, in particular the mascara compositions, may comprise an additional film former that is a cationic polymer. Suitable cationic polymers include, but are not limited to, Polyquaternium-4, Polyquaternium-5, Polyquaternium-6, Polyquaternium-7, Polyquaternium-10, Polyquaternium-22, Polyquaternium-37, Polyquaternium-47, or any combination thereof. Polyquaternium-7 is especially preferred. Polyquaternium-7 is a quaternary ammonium salt of a acrylamide/dimethyl diallyl ammonium chloride copolymer. Polyquaternium-7 is available as SALCARE® Super 7 (marketed by Ciba Specialty Chemicals, Inc.).

The compositions of the invention may optionally comprise other active and inactive ingredients typically associated with cosmetic and personal care products, including, but not limited to, excipients, fillers, emulsifying agents, antioxidants, surfactants, film formers, chelating agents, gelling agents, thickeners, emollient, humectants, moisturizers, vitamins, minerals, viscosity and/or rheology modifiers, sunscreens, keratolytics, depigmenting agents, retinoids, hormonal compounds, alpha-hydroxy acids, alpha-keto acids, anti-mycobacterial agents, antifungal agents, antimicrobials, antivirals, analgesics, lipidic compounds, anti-allergenic agents, H1 or H2 antihistamines, anti-inflammatory agents, anti-irritants, antineoplastics, immune system boosting agents, immune system suppressing agents, anti-acne agents, anesthetics, antiseptics, insect repellents, skin cooling compounds, skin protestants, skin penetration enhancers, exfollients, lubricants, fragrances, colorants, staining agents, depigmenting agents, hypopigmenting agents, preservatives, stabilizers, pharmaceutical agents, photostabilizing agents, and mixtures thereof. If present, the levels of such additional components should be judiciously selected so as not to adversely impact the ability of the emulsions to form superhydrophic films. Collectively, all such additional components should preferably comprise less than 5% by weight, more preferably less than 2% by weight, and more preferred still, less than 1% by weight of the total composition.

The combination of hydrophobic film forming polymer and hydrophobic pigment (e.g, hydrophobically-modified pigment, carbon black, etc.) typically will comprise from about 0.5% to about 99% of the cosmetic compositions. More, particularly, the combination of hydrophobic film forming polymer and hydrophobic pigment may comprise from about 1-5%, about 5-10%, about 10-15%, about 15-20%, about 20-25%, about 25-30%, about 30-35%, about 40-45%, about 45-50%, about 50-55%, about 55-60%, about 60-65%, about 65-70%, about 70-75%, about 75-80%, about 80-85%, about 85-90%, or about 90-95%, by weight of the cosmetic composition.

The hydrophobic pigment will typically comprise about 20% to about 95% of the weight of the dried film, by which is meant a film formed from the cosmetic composition after evaporation of any volatile components present. In various embodiments, the hydrophobic pigment will comprise from about 20-25%, about 25-30%, about 30-35%, about 40-45%, about 45-50%, about 50-55%, about 55-60%, about 60-65%, about 65-70%, about 70-75%, about 75-80%, or from about 80-85% of the dried film, on a weight basis. It is observed that the amount of hydrophobic pigment in the dried film is ideally adjusted toward the high end of the foregoing range in the case where the molecular weight of the film former is large (e.g., cellulosics), or where the film comprises high levels of non-volatile water-soluble or water-dispersible components which may coat or mask the pigment on the surface of the film. Preferably, the collective amount of non-volatile water-soluble or water-dispersible components in the fried film will be below about 35%, below about 30%, below about 25%, below about 20%, below about 15%, below about 10%, below about 5%, or below about 2.5%, based on the total weight of the dried film. In some embodiments, the superhydrophobic films will comprise less than 1% by weight of non-volatile water-soluble or water-dispersible components.

The hydrophobic pigment may comprise, consist essentially of, or consist of hydrophobically modified iron oxide. By "consist essentially of" hydrophobically modified iron oxide is meant that the presence of additional hydrophobic pigments is excluded to the extent that the presence of such additional hydrophobic pigments would have a measurable impact on the contact angle of the resultant film. In some embodiments, the hydrophobic pigment component may comprise more than about 5%, more than about 10%, more than about 15%, more than about 20%, more than about 25%, more than about 30%, more than about 35%, more than about 40%, more than about 45%, more than about 50%, more than about 55%, more than about 60%, more than about 65%, more than about 70%, more than about 75%, more than about 80%, more than about 85%, more than about 90%, or more than about 95% by weight hydrophobically-modified iron oxide.

The hydrophobic pigment may comprise, consist essentially of, or consist of carbon black. By "consist essentially of" carbon black is meant that the presence of additional hydrophobic pigments is excluded to the extent that the presence of such additional hydrophobic pigments would have a measurable impact on the contact angle of the resultant film. In some embodiments, the hydrophobic pigment component may comprise more than about 5%, more than about 10%, more than about 15%, more than about 20%, more than about 25%, more than about 30%, more than about 35%, more than about 40%, more than about 45%, more than about 50%, more than about 55%, more than about 60%, more than about 65%, more than about 70%, more than about 75%, more than about 80%, more than about 85%, more than about 90%, or more than about 95% by weight carbon black.

A mascara according to the invention may further comprise any of the customary ingredients for such a product, including those mascara ingredients listed in the International Cosmetic Ingredient Dictionary and Handbook, 12th Ed. (2008) at pages 3435-3438.

In one embodiment, the composition is formulated as a sunscreen comprising hydrophobically modified (i.e., surface treated) titanium dioxide or zinc oxide. The hydrophobically modified titanium dioxide or zinc oxide may comprise at from about 1% to about least about 15% of the total weight of the composition. The sunscreens will optionally comprise one or more organic UVA and/or UVB filters (hydrophobic or hydrophilic), although the levels of hydrophilic organic sunscreens in the emulsions should not be so high as to adversely impact the ability to form a superhydrophobic surface and the aggregate amount of such organic sunscreens will preferably be below about 10% by weight, more preferably below about 5% by weight. The sunscreens according to the invention will exhibit improved water-resistance as compared to conventional emulsion-based sunscreens.

The present composition may have one or more active sunscreens. Such sunscreen actives may be organic or inorganic and water-soluble or oil-soluble. Such actives include those for UVA and UVB protection (290 to 400 nanometer solar radiation). Such sunscreen actives include, but are not limited to, one or more of the following: dibenzoylmethane, oxybenzone, sulisobenzone, dioxybenzone, menthyl anthranilate, para aminobenzoic acid (PABA), octyl methoxycinnamate, DEA methoxycinnamate, octocrylene, drometrizole trisiloxane, octyl salicylate, homomenthyl salicylate, octyl dimethyl PABA, TEA salicylate, 4-methyl benzilidene camphor, octyl triazone, terephthalydiene dicamphor sulfonic acid, phenyl benzimidazole sulfonic acid, ethyl PABA, hydroxy methylphenyl benzotriazole, methylene bis-benzotriazoyltetramethylbutylphenol, bis-ethylhexyloxyphenol methoxyphenol triazine, titanium dioxide, zinc oxide, or any derivatives or any combinations thereof. Other useful sunscreen actives include those disclosed in U.S. Pat. No. 5,000,937. Preferred sunscreens include octylmethoxy cinnamate, octyl salicylate, octocrylene, avobenzone, benzophenone-3, and polysilicone-15 (Parsol slx).

In one embodiment, the compositions are applied to the skin, preferably the skin of the face. Such compositions may be formulated as a foundation, a blush, eyeshadow, etc. In another embodiment, the compositions are provided as a water-resistant, transfer-resistant lip product (e.g., a Lipstick or lip gloss), in which case the compositions are applied to the lips. In another embodiment, the compositions may be formulated as a nail polish. Color cosmetics, including foundations, mascaras, nail polishes, lip sticks, eye shadows, and the like will optionally comprise one or more additional colorants, including dyes, lakes, pigments, or combinations thereof.

In another embodiment, the compositions are applied to the hair (hair of the body, scalp, beard, mustache, eyelashes, etc.) and provide resistance against wetting. Thus, for example, the composition may be applied to the hair before swimming such that the hair does not become wet, or becomes only minimally wet, after submersion in water. By minimally wet is meant that the weight of the hair after submersion is increased by 100% or less, preferably by 50% or less, more preferably by 25% or less, and more preferred still by 10% or less as compared to the weight of the hair prior to submersion in water. Further, after one or two vigorous shakes of the hair, the hair will be essentially dry. By essentially dry is meant that the weight of the hair will be increased by less than about 5% or less than about 2.5% as compared to the weight of the hair before submersion. The foregoing may be tested using hair swatches treated with the inventive compositions. Likewise, the compositions may be applied to the hair of a pet, such as a dog, before swimming such that the pet is substantially dry immediately after swimming without the need for toweling off, etc., or to livestock so they are not wetted by snow, rain or mud.

Additional components may be incorporated as fillers or for various functional purposes as is customary in the cosmetic arts. However, while additional components consistent to formulate the above cosmetic compositions may be included, the inclusion of additional ingredients is limited to those ingredients which do not interfere with the formation of a superhydrophobic film.

EXAMPLES

Example 1

This Example compares three films comprising iron oxides hydrophobically-modified with perfluoroalkyl groups on the basis of their contact angle with a water droplet. In each case, the iron oxide pigment was Black Iron Oxides (INCI) surface-treated with Perfluorooctyl Triethoxysilane (INCI). The pigments differ with respect to particle size and degree of surface treatment. Table 1 provides the mean and median particle size, the percent surface treatment (ST), and the ratio of the percent surface treatment (ST) to the mean and median particle size for each of the iron oxide pigments used in this example.

Table 1.

| characteristics of perfluoroalkylsilane-treated iron oxide pigments | | | | | |
|---|---|---|---|---|---|
| | Particle size ($\mu$m) | | percent surface treatment[1] | ratio of percent surface treatment to particle size | |
| Sample | mean | median | (ST) | ST:mean | ST:median |
| A | 11.5 | 1.88 | 0.8 | 0.07 | 0.43 |
| B | 1.44 | 1.02 | 1.6 | 1.11 | 1.57 |
| C | 1.63 | 1.71 | 3 | 1.88 | 1.75 |
| A = Unipure black LC989 FS 8% (Sensient); B - Unipure black LC989 FS 1.6% (Sensient); and C = Black iron oxide FS 3% (Sensient); [1]defined as 100 times the weight ratio of Perfluorooctyl Triethoxysilane (INCI) to Iron Oxides (INCI). | | | | | |

The iron oxide pigments of Table 1 (Samples A, B, and C) were added to anhydrous dispersions of hydrophobic film formers in isododecane to prepare formulations 1A, 1B, and 1C, according to Table 2. All amounts listed in Table 2 are provided as weight percentage of the total composition.

Table 2.

| INCI name/description | 1A | 1B | 1C |
|---|---|---|---|
| isododecane[1] | 64 | 64 | 64 |
| isododecane and acrylates/dimethicone copolymer[2] | 6 | 6 | 6 |
| isododecane and ethylene/propylene/styrene copolymer and butylene/ethylene/styrene copolymer[3] | 20 | 20 | 20 |
| Pigment (Sample A) | 10 | -- | -- |
| Pigment (Sample B) | -- | 10 | -- |
| Pigment (Sample C) | -- | -- | 10 |
| total | 100 | 100 | 100 |
| [1]Permethyl A from Presperse; [2]KP 550 from Shin Etsu; [3]Versagel MD 1600 from Penreco. | | | |

Formulations 1A, 1B, and 1C were applied to glass slides and volatiles were allowed to evaporate to give a thin film. The contact angles of each film with a drop of water were measured using a Kruss Drop Shape Analysis System DSA 10 MK2. The contact angle was calculated via the instrument software using the circle fit method. The water volume (i.e., drop size) was set to 5 $\mu$l. The contact angles were measured to be 133° (1A). 138.6° (1B), and 143.1° (1C).

Table 3 shows the correlation coefficient ($r_{x,y}$) and the coefficient of determination ($r^2$) for the variables (x,y), where y is the measured contact angle and values x are the following independent variables: percent surface treatment (ST), median particle size (median), mean particle size (mean), ratio of ST to median particle size (ST:median), and ratio of ST to mean particle size (ST:mean).

Table 3,

| | ST | median | mean | ST:median | ST:mean |
|---|---|---|---|---|---|
| $r_{x,y}$ | 0,976 | -0.241 | -0.888 | 0.944 | 0.999 |

(continued)

|  | ST | median | mean | ST:median | ST:mean |
|---|---|---|---|---|---|
| $r^2$ | 0.953 | 0.058 | 0.789 | 0,891 | 0.999 |

As shown in Table 3, the ratio ST:mean (i.e., the ratio of percent surface treatment to mean particle size in microns) for iron oxide pigments surface treated with Perfluorooctyl Triethoxysilane (INCI) is a better predictor of film hydrophobicity than the other independent variables.

Example 2

This Example compares three films comprising iron oxides hydrophobically-modified with alkyl chains on the basis of their contact angle with a water droplet. In each case, the iron oxide pigment comprised Black Iron Oxides (INCI) surface-treated with Triethoxycaprylylsilane (INCI). The pigments differ with respect to particle size and degree of surface treatment. Table 4 provides the mean particle size (mean), the percent surface treatment (ST), and the ratio of the percent surface treatment to the mean particle size (ST:mean) for each of the iron oxide pigments used in this example.

Table 4.

| characteristics of alkylsilane-treated iron oxide pigments | | | |
|---|---|---|---|
| Sample | mean particle size ($\mu$m) | percent surface treatment[1] (ST) | ST:mean |
| D | 0.506 | 1 | 1.98 |
| E | 0.444 | 1 | 2.25 |
| F | 0.765 | 2 | 2.61 |
| D = Black iron oxide AS # 4023 (Sensient), a triethoxycaprylylsilane treated iron oxide pigment; E = Covalumine black AS (Sensient), a triethoxycaprylylsilane treated iron oxide and alumina pigment; and F = Black NF 11S2 (Kobo), a triethoxycaprylylsilane treated iron oxide pigment; [1]defined as 100 times the weight ratio of triethoxycaprylylsilane to metal oxides. | | | |

The alkylsilane-treated iron oxide pigments of Table 4 (Pigment Samples D, E, and F) were added to anhydrous dispersions of hydrophobic film formers in isododecane to prepare formulations 2D, 2E, and 2F, according to Table 5. All amounts listed in Table 2 are provided as weight percentage of the total composition.

Table 5.

| INCI name/description | 2D | 2E | 2F |
|---|---|---|---|
| isododecane[1] | 64 | 64 | 64 |
| isododecane and acrylates/dimethicone copolymer[2] | 6 | 6 | 6 |
| isododecane and ethylene/propylene/styrene copolymer and butylene/ethylene/styrene copolymer[3] | 20 | 20 | 20 |
| Pigment (Sample D) | 10 | -- | -- |
| Pigment (Sample E) | -- | 10 | -- |
| Pigment (Sample F) | -- | -- | 10 |
| total | 100 | 100 | 100 |
| [1]Permethyl A from Presperse; [2]KP 550 from Shin Etsu; [3]Versagel MD 1600 from Penreco. | | | |

Formulations 2D, 2E, and 2F were applied to glass slides and volatiles were allowed to evaporate to give a thin film. The contact angles of each film with a drop of water were measured using a Kruss Drop Shape Analysis System DSA 10 MK2. The contact angle was calculated via the instrument software using the circle fit method, The water volume (i.e., drop size) was set to 5 $\mu$l. The contact angles were measured to be 113.2° (2D), 114 ° (2E), and 142,8° (2F).

As with the perfluoroalkylsilane-treated iron oxide pigments of Example 1, the ratio ST:mean (ratio of percent surface treatment to mean particle size in microns) was strongly correlated with hyrophobicity of the resultant films with the highest ST:mean pigment providing the most hydrophobic film. Although in this case the percent surface treatment (ST) alone and the mean particle size (mean) alone produced greater $r_{x,y}$ and $r^2$ values than the ratio ST:mean, the significance of this finding is discounted, in part because of the limited data set and in part because pigment Sample E (Covalumine black AS from Sensient) comprises 79% by weight alumina and 20% by weight iron oxide, whereas the pigments of Samples D and F comprise only iron oxide as the metal oxide component.

Nevertheless, it is established that alkylsilane-treated iron oxide pigments having a ratio of percent surface treatment to mean particle size in microns (ST:mean) greater than about 2.5 are capable of providing a superhydrophobic film.

Example 3

This example provides a composition for imparting a superhydrophobic film comprising carbon black (D&C Black # 2) and hydrophobic film formers in an anhydrous vehicle. The composition has the formulation provided in Table 6.

Table 6.

| INCI name/description | Sample 3 |
| --- | --- |
| isododecane[1] | 64 |
| isadodecane and acrylates/dimethicone copolymer[2] | 6 |
| isododecane and ethylene/propylene/styrene copolymer and butylene/ethylene/styrene copolymer[3] | 20 |
| D&C Black # 2[4] | 10 |
| total | 100 |
| [1]Permethyl A from Presperse: [2]KP 550 from Shin Etsu; [3]Versagel MD 1600 from Penreco; [4]Unipure black LC 902 from Sensient. | |

A film was prepared by depositing the composition of Table 6 on a glass slide and permitting the volatiles to evaporate. The contact angle with a drop of water was measured to be 148.2°.

Example 4

This example demonstrates the synergistic improvement in superhydrophobicity obtainable using a mixture of carbon black pigment and alkylsilane-treated iron oxide pigment. Formulations having varying ratios of carbon black pigment to iron oxide pigment were prepared according to Table 7.

Table 7.

| INCI name/description | Example No. | | | | |
| --- | --- | --- | --- | --- | --- |
| | 2D | 4H | 4I | 4J | 3 |
| isododecane[1] | 64 | 64 | 64 | 64 | 64 |
| isododecane and acrylates/dimethicone copolymer[2] | 6 | 6 | 6 | 6 | 6 |
| isododecane and ethylene/propylene/styrene copolymer and butylene/ethylene/styrene copolymer[3] | 20 | 20 | 20 | 20 | 20 |
| Pigment (Sample D) | 10 | 7.5 | 5.0 | 2.5 | -- |
| D&C Black # 2[4] | -- | 2.5 | 5.0 | 7.5 | 10 |
| total | 100 | 100 | 100 | 100 | 100 |
| Permethyl A from Presperse; [2]KP 550 from Shin Etsu; [3]Versagel MD 1600 from Penreco; [4]Unipure black LC 902 from Sensient. | | | | | |

As discussed in Example 2, the contact angle with water for the sample comprising 10% by weight alkylsilane-treated iron oxide (Sample 2D) was found to be 113.2°. The sample comprising 10% by weight carbon black (D&C Black # 2) was shown in Example 3 to have a contact angle with water of 148.2°. These values were used to predict the contact angle of samples having 7.5% by weight pigment Sample D and 2.5% by weight D&C Black # 2; 5% by weight pigment Sample D and 5% by weight D&C Black # 2; and 2.5% by weight pigment Sample D and 7.5% by weight D&C Black # 2, assuming that the contribution of each pigment to the contact angle is additive. The results are plotted in Figure 1 as indicated by the dashed line and marker symbol (◊).

Films were prepared from Samples 4H, 4I, and 4J as discussed above, and the contact angle of each film was measured as above to be 144.5° (4H), 147° (4I), 145.8° (4J). These measured values are plotted in Figure 1 as indicated by the solid line and marker symbol (⊏), In each case, the measured contact angle is substantially greater than the predicted value, indicating a synergy between carbon black and alkylsilane-treated iron oxide with respect to the super-rhydrophobicity of the films.

Example 5

This example demonstrates the synergistic improvement in superhydrophobicity obtainable using a mixture of carbon black pigment and perfluoroalkylsilane-treated iron oxide pigment. Formulations having varying ratios of carbon black pigment to iron oxide pigment were prepared according to Table 8.

Table 8.

| INCI name/description | Example No. | | | | |
|---|---|---|---|---|---|
| | 1A | 5K | 5L | 5M | 3 |
| isododecane[1] | 64 | 64 | 64 | 64 | 64 |
| isododecane and acrylates/dimethicone copolymer[2] | 6 | 6 | 6 | 6 | 6 |
| isododecane and ethylene/propylene/styrene copolymer and butylene/ethylene/styrene copolymer[3] | 20 | 20 | 20 | 20 | 20 |
| Pigment (Sample A) | 10 | 7.5 | 5.0 | 2.5 | -- |
| D&C Black # 2[(4)] | -- | 2.5 | 5.0 | 7.5 | 10 |
| total | 100 | 100 | 100 | 100 | 100 |
| [1]Permethyl A from Presperse; [2]KP 550 from Shin Etsu; [3]Versagel MD 1600 from Penreco; [4]Unipure black LC 902 from Sensient. | | | | | |

As discussed in Example 1, the contact angle with water for the sample comprising 10% by weight perfluoroalkylsilane-treated iron oxide (Sample 1A) was found to be 133°, The sample comprising 10% by weight carbon black (D&C Black # 2) was shown in Example 3 to have a contact angle with water of 148.2°. These values were used to predict the contact angle of samples having 7.5% by weight pigment Sample A and 2.5% by weight D&C Black # 2; 5% by weight pigment Sample A and 5% by weight D&C Black # 2; and 2.5% by weight pigment Sample A and 7.5% by weight D&C Black # 2, assuming that the contribution of each pigment to the contact angle is additive. The results are plotted in Figure 2 as indicated by the dashed line and marker symbol (◊).

Films were prepared from Samples 5K, 5L, and 5M as discussed above, and the contact angle of each film was measured as above to be 148,1° (5K), 149.6° (5L), 150.3° (5M), These measured values are plotted in Figure 2 as indicated by the solid line and marker symbol (⊐). In each case, the measured contact angle is substantially greater than the predicted value, indicating a synergy between carbon black and perfluoroalkylsilane-treated iron oxide with respect to the superhydrophobicity of the films.

The invention described and claimed herein is limited by the scope of the claims but is not to be limited in scope by the specific embodiments herein disclosed since these embodiments are intended as illustrations of several aspects of the invention. Any equivalent embodiments are intended to be within the scope of this invention, but only in as far as they are covered by the claims.

A cosmetic composition for imparting a superhydrophobic film on an integument may comprise the following features, either alone or in any combination: - (a) one or more hydrophobic film formers, (b) carbon black; wherein the weight ratio of said one or more hydrophobic film formers to said carbon black is from about 1:10 to about 10:1; and wherein the aggregate weight percentage of all non-volatile water-soluble or water-dispersible organic constituents in said composition is less than 15%, based on the entire weight of the composition; said composition being capable of providing a film on a surface which, after evaporation of volatile constituents, is characterized by a contact angle with water greater than about 140°;

- on or more hydrophobically modified iron oxide pigments;
- one or more hydrophobically-modified iron oxide pigments comprise at least one of an alkylsilane-treated iron oxide pigment, at least one perfluoroalkylsilane-treated iron oxide pigment, or a mixture thereof;
- one or more hydrophobically-modified iron oxide pigments is selected from the group consisting of trialkyoxyalkylsilane treated iron oxide pigments, perfluoroalkyl trialkyoxysilane-treated iron oxide pigments, and mixtures thereof;
- said trialkyoxyalkylsilane-treated iron oxide pigment is a Triethoxycaprylylsilane-treated iron oxide pigment, and said perfluoroalkyl trialkyoxysilane-treated iron oxide pigment is a Perfluorooctyl Triethoxysilane-treated iron oxide pigment;
- one or more hydrophobic film formers comprise a film former selected from the groups consisting of (alkyl)acrylates, polyurethanes, fluoropolymers, silicones, and copolymers, thereof;
- one or more hydrophobic film formers comprises an acrylates/dimethicone copolymer;
- one or more hydrophobic film formers comprises a copolymer of two or more blocks selected from styrene (S), alkylstyrene (AS), ethylene/butylene (EB), ethylene/propylene (EP), butadiene (B), isoprene (I), acrylate (A) and methacrylate (MA);
- the weight ratio of said one or more hydrophobic film formers to the hydrophobically modified iron oxide pigments plus the carbon black is from about 1:5 to about 2:1;
- the aggregate weight percentage of all non-volatile water-soluble or water-dispersible organic constituents in said composition is less than 2%;
- said carbon black has a mean particle size between about 0.1 $\mu$m and about 1 $\mu$m and surface area between about 50 and about 500 m$^2$/g;
- the weight ratio of said one or more hydrophobic film formers to said carbon black is from about 1:10 to about 2:1;
- the weight percentage of all polyols is collectively below 1%,

## Claims

1. A cosmetic composition for imparting a superhydrophobic film on an integument comprising:

   (a) one or more hydrophobic film formers, and
   (b) a combination of hydrophobic pigments comprising: (i) one or more hydrophobically-modified iron oxide pigments; and (ii) a carbon black powder; wherein the weight ratio of said one or more hydrophobically-modified iron oxide pigments to said carbon black powder is being between 1:10 to 10:1, and wherein the one or more hydropobically-modified iron oxide pigment is selected from the group consisting of alkylsilane-treated iron oxide pigments, and perfluoroalkylsilane-treated iron oxide pigments and mixtures thereof;

   wherein the weight ratio of said one or more hydrophobic film formers to said combination of hydrophobic pigments is from 1:10 to 5:1; and wherein the aggregate weight percentage of all non-volatile water-soluble or water-dispersible organic constituents in said composition is less than 15%, based on the entire weight of the composition; said composition is set to provide a film on a surface which, after evaporation of any volatile constituents present, has a contact angle with water greater than 140°.

2. The composition according to claim 1, wherein the carbon black powder has a mean particle size between 0.01 $\mu$m and 1 $\mu$m and/or a surface area between 200 and 260 m$^2$/g.

3. The composition according to claim 1, wherein said pigment comprises one or more trialkyoxyalkylsilane treated iron oxide pigments.

4. The composition according to claim 3, wherein said pigment is a triethoxycaprylylsilane treated iron oxide pigment having a ratio of percent surface treatment (ST) to mean particle size greater than 2.5, and wherein with $W_1$ as weight of the surface treatment agent triethoxycaprylylsilane and with $W_2$ as weight of the iron oxide pigment the percent surface treatment (ST) is defined as:

$$ST = 100 \text{ x} \left( \frac{W_1}{W_2 + W_1} \right).$$

5. The composition according to claim 1, wherein said pigment comprises one or more perfluoroalkyl trialkyoxysilane treated iron oxide pigments.

6. The composition according to claim 5, wherein said pigment is a Perfluorooctyl Triethoxysilane treated iron oxide pigment having a ratio of percent surface treatment to mean particle size greater than 1.5, and wherein with $W_1$ as weight of the surface treatment agent triethoxycaprylylsilane and with $W_2$ as weight of the iron oxide pigment the percent surface treatment (ST) is defined as:

$$ST = 100 \text{ x} \left( \frac{W_1}{W_2 + W_1} \right).$$

7. The composition according to claim 1, wherein said one or more hydrophobic film formers comprise a film former selected from the group consisting of (alkyl)acrylates, polyurethanes, fluoropolymers, silicones, and copolymers thereof.

8. The composition according to claim 7, wherein said one or more hydrophobic film formers comprises an copolymer of acrylate and dimethicone.

9. The composition according to claim 1, wherein said one or more hydrophobic film formers comprises a copolymer of two or more blocks selected from styrene (S), alkylstyrene (AS), ethylene/butylene (EB), ethylene/propylene (EP), butadiene (B), isoprene (I), acrylate (A) and methacrylate (MA).

10. The composition according to claim 1, wherein the iron oxide pigment is alkylsilane-treated and the weight ratio of said one or more hydrophobic film formers to said one or more alkylsilane-treated iron oxide pigments is from 1:10 to 2:1.

11. The composition according to claim 1, wherein the iron oxide pigment is fluorosilane-treated and the weight ratio of said one or more hydrophobic film formers to said one or more perfluoroalkylsilane-treated iron oxide pigments is from 1:5 to 2:1.

12. The composition according to claim 1, wherein the aggregate weight percentage of all non-volatile water-soluble or water-dispersible organic constituents in said composition is less than 2%.

13. The composition according to claim 1, wherein the weight percentage of all polyols is collectively below 1%.

14. A method for imparting a superhydrophobic film to the eyelashes comprising applying thereto the composition according to claim 1 and allowing any volatile constituents present to evaporate, thereby forming a hydrophobic film **characterized by** a contact angle with a water droplet of at least 140°.

**Patentansprüche**

1. Kosmetische Zusammensetzung zum Erzeugen eines superhydrophoben Films auf einem Integument, umfassend:

(a) einen oder mehrere hydrophobe Filmbildner, und
(b) eine Kombination aus hydrophoben Pigmenten, umfassend: (i) ein oder mehrere hydrophob modifizierte Eisenoxidpigmente; und (ii) ein Rußpulver; wobei das Gewichtsverhältnis des einen oder der mehreren hydrophob modifizierten Eisenoxidpigmente zu dem Rußpulver zwischen 1:10 und 10:1 liegt und wobei das eine oder die mehreren hydrophob modifizierten Eisenoxidpigmente ausgewählt sind aus der Gruppe, bestehend aus Alkylsilanbehandelten Eisenoxidpigmenten und Perfluoralkylsilan-behandelten Eisenoxidpigmenten und Gemischen davon;

wobei das Gewichtsverhältnis des einen oder der mehreren hydrophoben Filmbildner zu der Kombination aus hydrophoben Pigment bei 1:10 bis 5:1 liegt und wobei der Gesamtgewichtsanteil aller nichtflüchtigen wasserlöslichen oder wasserdispergierbaren organischen Bestandteile in der Zusammensetzung weniger als 15 % bezogen auf das Gesamtgewicht der Zusammensetzung beträgt;
wobei die Zusammensetzung derart eingerichtet ist, dass sie einen Film auf einer Oberfläche bereitstellt, der nach Verdunstung vorhandener flüchtiger Bestandteile einen Kontaktwinkel zu Wasser von größer als 140° aufweist.

2. Zusammensetzung nach Anspruch 1, wobei das Rußpulver eine mittlere Partikelgröße zwischen 0,01 $\mu$m und 1 $\mu$m und/oder eine spezifische Oberfläche zwischen 200 und 260 m$^2$/g aufweist.

3. Zusammensetzung nach Anspruch 1, wobei das Pigment ein oder mehrere Trialkyoxyalkylsilan-behandelte Eisenoxidpigmente umfasst.

4. Zusammensetzung nach Anspruch 3, wobei das Pigment ein Triethoxycaprylylsilan-behandeltes Eisenoxidpigment mit einem Verhältnis von prozentualer Oberflächenbehandlung (ST) zu mittlerer Partikelgröße von größer als 2,5 aufweist und wobei mit $W_1$ als Gewicht des Oberflächenbehandlungsmittels Triethoxycaprylylsilan und mit $W_2$ als Gewicht des Eisenoxidpigments die prozentuale Oberflächenbehandlung (ST) definiert ist als:

$$ST = 100 \text{ x} \left( \frac{W_1}{W_2 + W_1} \right).$$

5. Zusammensetzung nach Anspruch 1, wobei das Pigment ein oder mehrere Perfluoralkyltrialkyoxysilan-behandelte Eisenoxidpigmente umfasst.

6. Zusammensetzung nach Anspruch 5, wobei das Pigment ein Perfluoroctyltriethoxysilan-behandeltes Eisenoxidpigment mit einem Verhältnis von prozentualer Oberflächenbehandlung zu mittlerer Partikelgröße von größer als 1,5 ist und wobei mit $W_1$ als Gewicht des Oberflächenbehandlungsmittels Triethoxycaprylylsilan und mit $W_2$ als Gewicht des Eisenoxidpigments die prozentuale Oberflächenbehandlung (ST) definiert ist als:

$$ST = 100 \text{ x} \left( \frac{W_1}{W_2 + W_1} \right).$$

7. Zusammensetzung nach Anspruch 1, wobei der eine oder die mehreren hydrophoben Filmbildner einen Filmbilder umfassen, der ausgewählt ist aus der Gruppe, bestehend aus (Alkyl)acrylaten, Polyurethanen, Fluorpolymeren, Silikonen und Copolymeren davon.

8. Zusammensetzung nach Anspruch 7, wobei der eine oder die mehreren hydrophoben Filmbildner ein Copolymer aus Acrylat und Dimeticon umfassen.

9. Zusammensetzung nach Anspruch 1, wobei der eine oder die mehreren hydrophoben Filmbildner ein Copolymer aus zwei oder mehr Blöcken umfassen, die ausgewählt sind aus Styrol (S), Alkylstyrol (AS), Ethylen/Butylen (EB), Ethylen/Propylen (EP), Butadien (B), Isopren (I), Acrylat (A) und Methacrylat (MA).

10. Zusammensetzung nach Anspruch 1, wobei das Eisenoxidpigment Alkylsilanbehandelt ist und das Gewichtsverhältnis des einen oder der mehreren hydrophoben Filmbildner zu dem einen oder den mehreren Alkylsilanbehandelten Eisenoxidpigmenten bei 1:10 bis 2:1 liegt.

11. Zusammensetzung nach Anspruch 1, wobei das Eisenoxidpigment Fluorsilanbehandelt ist und das Gewichtsverhältnis des einen oder der mehreren hydrophoben Filmbildner zu dem einen oder den mehreren Perfluoralkylsilanbehandelten Eisenoxidpigmenten bei 1:5 bis 2:1 liegt.

12. Zusammensetzung nach Anspruch 1, wobei der Gesamtgewichtsanteil aller nichtflüchtigen wasserlöslichen oder wasserdispergierbaren organischen Bestandteile in der Zusammensetzung weniger als 2 % beträgt.

13. Zusammensetzung nach Anspruch 1, wobei der Gewichtsanteil aller Polyole zusammen unter 1 % liegt.

14. Verfahren zum Erzeugen eines superhydrophoben Films auf den Wimpern, umfassend Auftragen der Zusammensetzung nach Anspruch 1 darauf und Verdunstenlassen vorhandener flüchtiger Bestandteile, wodurch ein hydrophober Film gebildet wird, der durch einen Kontaktwinkel zu einem Wassertropfen von wenigstens 140° gekennzeichnet ist.

**Revendications**

1. Composition cosmétique destinée à créer un film superhydrophobe sur un tégument, comprenant :

   (a) un ou plusieurs agents filmogènes hydrophobes et
   (b) une combinaison de pigments hydrophobes comprenant : (i) un ou plusieurs pigments à l'oxyde de fer modifiés pour être hydrophobes et (ii) une poudre de noir de carbone, le rapport de poids entre lesdits un ou plusieurs pigments à l'oxyde de fer modifiés pour être hydrophobes et ladite poudre de noir de carbone étant compris entre 1 pour 10 et 10 pour 1 et les un ou plusieurs pigments à l'oxyde de fer modifiés pour être hydrophobes étant choisis parmi le groupe comprenant des pigments à l'oxyde de fer traités avec un alkylsilane et des pigments à l'oxyde de fer traités avec un perfluoroalkylsilane et des mélanges de ceux-ci ;

   dans laquelle le rapport de poids desdits un ou plusieurs agents filmogènes hydrophobes par rapport à ladite combinaison de pigments hydrophobes est de 1 pour 10 à 5 pour 1, et
   dans laquelle le pourcentage en poids total de tous les composants organiques non volatils hydrosolubles ou hydrodispersibles dans ladite composition est inférieur à 15 % du poids total de la composition,
   ladite composition étant ajustée afin de produire sur une surface qui, après évaporation des composants volatils éventuellement présents, a un angle de contact avec l'eau supérieur à 140°.

2. Composition selon la revendication 1, dans laquelle la poudre de noir de carbone a une taille de particules moyenne comprise entre 0,01 $\mu$m et 1 $\mu$m et/ou une aire de surface comprise entre 200 et 260 m$^2$/g.

3. Composition selon la revendication 1, dans laquelle ledit pigment comprend un ou plusieurs pigments à l'oxyde de fer traités avec du trialkyoxyalkylsilane.

4. Composition selon la revendication 3, dans laquelle ledit pigment est un pigment à l'oxyde de fer traité avec du triéthoxycaprylylsilane dont le rapport entre le pourcentage de traitement de surface (ST) et la taille de particules moyenne est supérieur à 2,5, et dans laquelle, W1 étant le poids du triéthoxycaprylylsilane servant au traitement de la surface et W2 le poids du pigment à l'oxyde de fer, le pourcentage de traitement de la surface (ST) est défini par :

$$ST = 100 \times \left( \frac{W_1}{W_2 + W_1} \right).$$

5. Composition selon la revendication 1, dans laquelle ledit pigment comprend un ou plusieurs pigments à l'oxyde de fer traités avec du perfluoroalkyl-trialkyoxysilane.

6. Composition selon la revendication 5, dans laquelle ledit pigment est un pigment à l'oxyde de fer traité avec du perfluorooctyl-triéthoxysilane ayant un rapport entre le pourcentage de traitement de la surface et la taille moyenne de particules supérieur à 1,5, et dans laquelle, W1 étant le poids du triéthoxycaprylylsilane servant au traitement de la surface et W2 le poids du pigment à l'oxyde de fer, le pourcentage de traitement de la surface (ST) est défini par :

$$ST = 100 \times \left( \frac{W_1}{W_2 + W_1} \right).$$

7. Composition selon la revendication 1, dans laquelle lesdits un ou plusieurs agents filmogènes hydrophobes comprennent un agent filmogène choisi parmi le groupe comprenant des acrylates (d'alkyle), des polyuréthanes, des fluoropolymères, des silicones et des copolymères de ceux-ci.

8. Composition selon la revendication 7, dans laquelle lesdits un ou plusieurs agents filmogènes hydrophobes comprennent un copolymère d'acrylate et de diméthicone.

9. Composition selon la revendication 1, dans laquelle lesdits un ou plusieurs agents filmogènes hydrophobes comprennent un copolymère de deux ou plusieurs blocs choisis parmi le styrène (S), l'alkylstyrène (AS), l'éthylène/butylène (EB), l'éthylène/propylène (EP), le butadiène (B), l'isoprène (I), l'acrylate (A) et le méthacrylate (MA).

10. Composition selon la revendication 1, dans laquelle le pigment à l'oxyde de fer est traité avec un alkylsilane et le rapport de poids desdits un ou plusieurs agents filmogènes hydrophobes avec lesdits un ou plusieurs pigments à l'oxyde de fer traités avec un alkylsilane est compris entre 1 pour 10 et 2 pour 1.

11. Composition selon la revendication I, dans laquelle le pigment à l'oxyde de fer est traité avec du fluorosilane et le rapport de poids desdits un ou plusieurs agents filmogènes hydrophobes avec lesdits un ou plusieurs pigments à l'oxyde de fer traités avec du perfluoroalkylsilane est compris entre 1 pour 5 et 2 pour 1.

12. Composition selon la revendication 1, dans laquelle le pourcentage de poids total de tous les composants organiques non volatils hydrosolubles ou hydrodispersibles dans ladite composition est inférieur à 2 %.

13. Composition selon la revendication 1, dans laquelle le pourcentage en poids total de tous les polyols est inférieur à 1 %.

14. Procédé pour créer un film superhydrophobe sur les cils comprenant l'application sur ceux-ci de la composition selon la revendication 1 et l'évaporation des composants volatils éventuellement présents pour former un film **caractérisé par** un angle de contact avec les gouttelettes d'eau d'au moins 140°.

**Synergistic Improvement in Superhydrophobicity Between
Carbon Black and Alkylsilane-Treated Iron Oxide Pigments**

Figure 1

**Synergistic Improvement in Superhydrophobicity Between Carbon Black and
Perfluoroalkylsilane-Treated Iron Oxide Pigments**

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61033536 B
- US 6683126 B
- US 6852389 B
- US 6946170 B
- US 7056845 B
- US 6800354 B
- US 5500216 A
- JP 2001181136 B
- US 7037515 B, Kalafsky
- US 6685952 B, Ma
- US 6464969 B, De La Poterie
- US 6264933 B, Bodelin
- US 6683125 B, Keller
- US 5911980 A, Samour
- US 7150878 B, Gonzalez
- US 3393155 A, Schutte
- US 2705206 A, Wagner
- US 20060110541 A, Russell
- US 20060110542 A, Dietz
- US 6315990 B, Farer
- EP 1640419 A
- US 20050000531 A
- US 4781917 A
- US 4122029 A
- US 5688831 A
- US 5000937 A

**Non-patent literature cited in the description**

- **TODD et al.** Volatile Silicone Fluids for Cosmetics. *Cosmetics and Toiletries,* 1976, vol. 91, 27-32
- International Cosmetic Ingredient Dictionary and Handbook. 2008, 3435-3438